# EUROPEAN PATENT APPLICATION

(11) **EP 1 405 870 A1**
(43) Date of publication of application: **07.04.2004**
(21) Application number: 02705412.1
(22) Date of filing: 20.03.2002
(51) Int. Cl.: C08G 79/00, C07D 487/22

(54) **MONOMERS HAVING ROTAXANE STRUCTURE AND POLYMERS AND PROCESSES FOR PRODUCTION OF BOTH**

(30) Priority: 30.05.2001 JP 2001163488; 30.05.2001 JP 2001163477
(71) Applicant: National Institute of Advanced Industrial Science and Technology, Tokyo 100-0013 (JP)
(72) Inventor: ASAKAWA, Masumi, Nat. Inst. of Adv. Ind. Sci.&Tec., Tsukuba-shi, Ibaraki 305-0046 (JP); YAMANISHI, Hiroko, Nat. Inst. of Adv.Ind. Sci.&Tec, Tsukuba-shi, Ibaraki 305-0046 (JP); SHIMIZU, Toshimi, Nat. Inst. of Adv. Ind. Sci.&Tec, Tsukuba-shi, Ibaraki305-0046 (JP)
(74) Representative: Luderschmidt, Schüler & Partner GbR
(86) International application number: PCT/JP2002/002714
(87) International publication number: WO 2002/098956

(57) **Abstract**

Disclosed is a monomer represented by the following general formula (Ia): 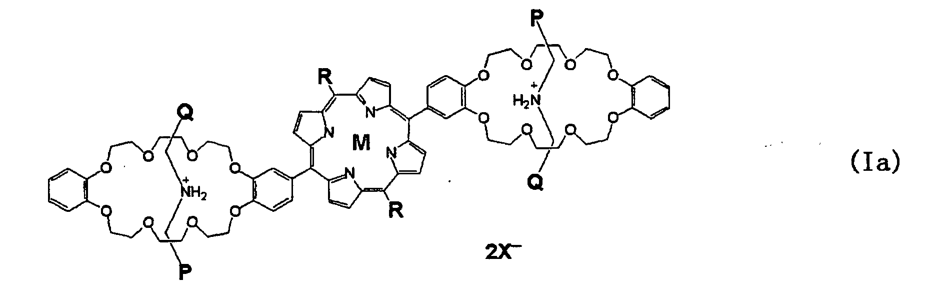 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom. The monomer can give a polymer via metal coordination interaction and hydrogen bonding.

## Description

### Technical Field:

The present invention relates to a monomer based on a rotaxane structure, to a process of preparing the monomer, to a polymer and to a process of preparing the polymer.

### Background Art:

Very recently, there was a report concerning the results of studies on polymers polymerized by hydrogen bonding (Science, vol. 278, page 1601 (1997).

It has been theoretically predicted that there is a limitation in the technology of semiconductors relying upon physical properties of silicon, i.e. the technology has been shown to be reaching its technical limitation within more than ten years. Since then, compliances are suddenly lectured for exploring and utilizing new materials which have a function as electronic parts. Thus, studies have been made on organic molecules and organic polymers. As one field of such application, there is a report concerning studies on synthesis of molecular wires containing a multiplicity of porphyrin molecules connected in series by covalent bonds (Angewante Chemie International Edition of English, vol. 39, page 1458 (2000)).

Synthesis of rotaxanes in which macrocycle molecules and linear molecules are mechanically connected has been rapidly developed in those several years. The present inventors have made an invention (Japanese patent application No. 2000-71252) by interest in the synthesis thereof. In this field, a number of studies have been made on application to molecular elements by utilization of characteristic structure of the rotaxane (Science, vol. 285, page 391 (1999).

When an electronic part is produced from a rotaxane in which macrocycle molecules and linear molecules are mechanically connected, it is possible to respond to an outside signal such as electricity, light or heat without changing its shape. This provides a significant merit in view of the assembly of electronic parts in a nanometer scale.

A molecular wire is required to extend between electronic parts. To achieve this purpose, it is necessary to construct a molecular wire having a length sufficient to extend between electronic parts. With a method in which covalent bonds are utilized to connect molecules, it is very difficult to prepare molecular wires. Conventional rotaxanes also have difficulties in producing polymers and cannot produce molecular wires.

In view of the above background, the present inventors have thought that a more intelligent molecular machine may be constructed by blending porphyrin which is anticipated as being promising as a molecular wire with a rotaxane which is anticipated as a molecular device. The above idea has been developed to an attempt to utilize non-covalent bonding for uniting the rotaxane and porphyrin. Thus, the present inventors have made a study on polymerization of organic compounds by non-covalent links with an anticipation for stepping up to the realization of a molecular computer.

It is an objective problem of the present invention to provide a monomer for preparing a polymer, a polymer obtainable from such a monomer, and a process of preparing the monomer and polymer.

The present inventors have made an earnest study with a view toward developing a polymer constructed by non-covalent links utilizing metal coordination interaction and hydrogen bonding interaction and have completed the present invention.

Thus, in accordance with the present invention, there are provided the following inventions:
1. A monomer represented by the following general formula (Ia): 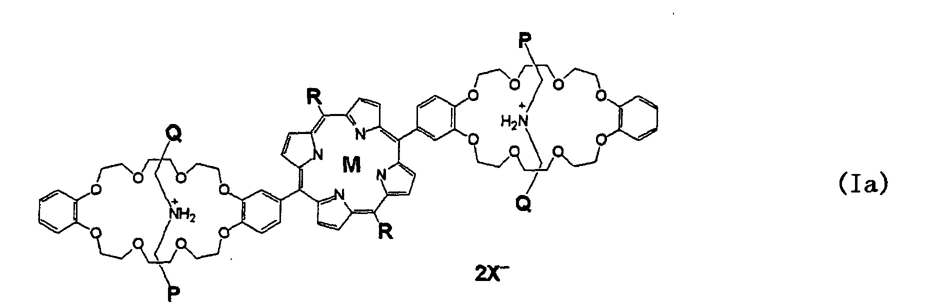 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.
2. A monomer represented by the following general formula (Ib): 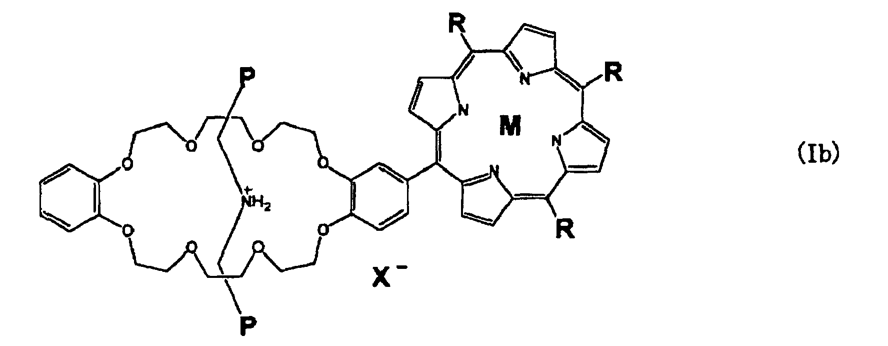 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.
3. A compound represented by the following general formula (IIIa): 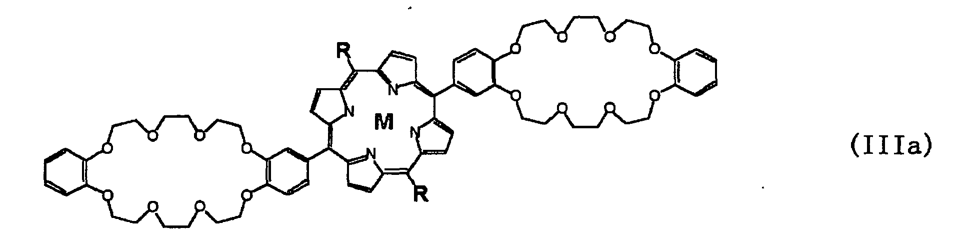 wherein M represents a transition metal coordinatable with the four nitrogen atoms and two additional ligands and R represents a hydrocarbyl group selected from aliphatic hydrocarbon groups and aromatic hydrocarbon groups.
4. A compound represented by the following general formula (IIIb): 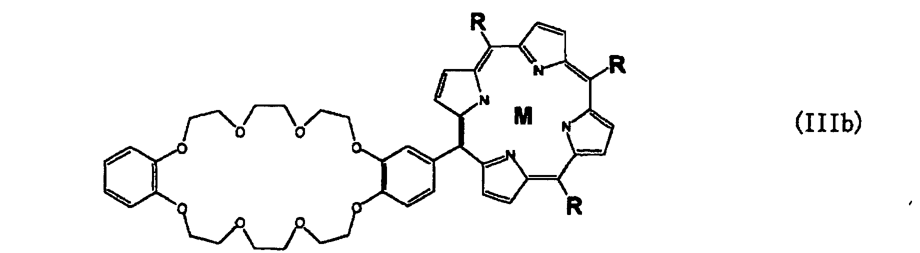 wherein M represents a transition metal coordinatable with the four nitrogen atoms and two additional ligands and R represents a hydrocarbyl group selected from aliphatic hydrocarbon groups and aromatic hydrocarbon groups.
5. A secondary ammonium salt represented by the following general formula (IVa): 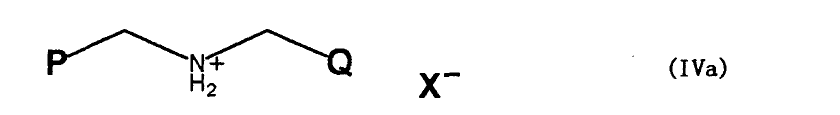 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups and X represents an arbitrary anion atom.
6. A secondary ammonium salt represented by the following general formula (IVb): 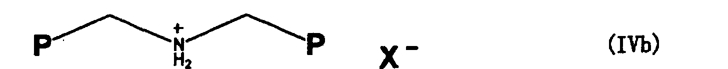 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, and X represents an arbitrary anion atom.
7. A process for the preparation of a monomer represented by the following general formula (Ia): 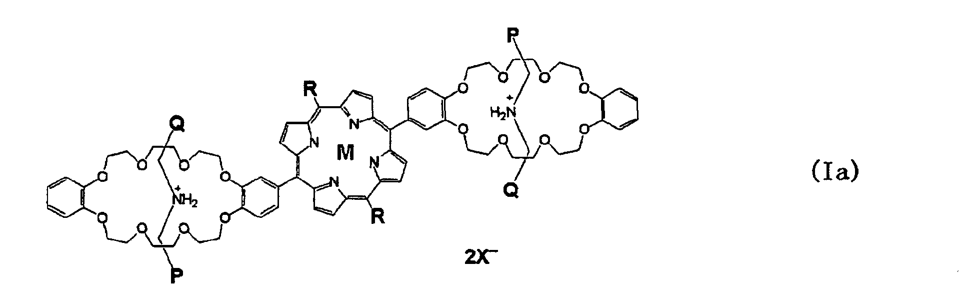 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom, said process comprising reacting a compound represented by the following general formula (IIIa): 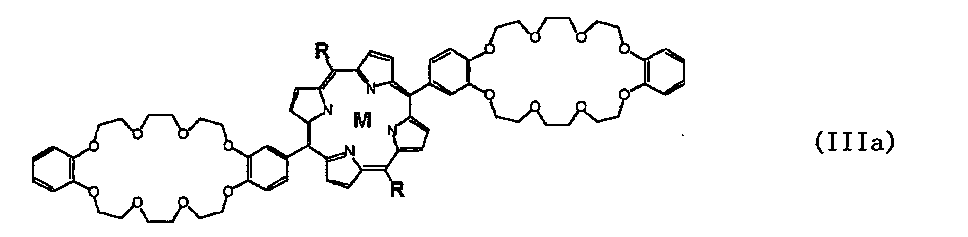 wherein M represents a transition metal coordinatable with the four nitrogen atoms and two additional ligands and R represents a hydrocarbyl group selected from aliphatic hydrocarbon groups and aromatic hydrocarbon groups, with a secondary ammonium salt represented by the following general formula (IVa): 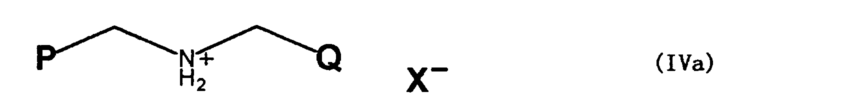 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups and X represents an arbitrary anion atom, in a solvent.
8. A process for the preparation of a monomer represented by the following general formula (Ib): 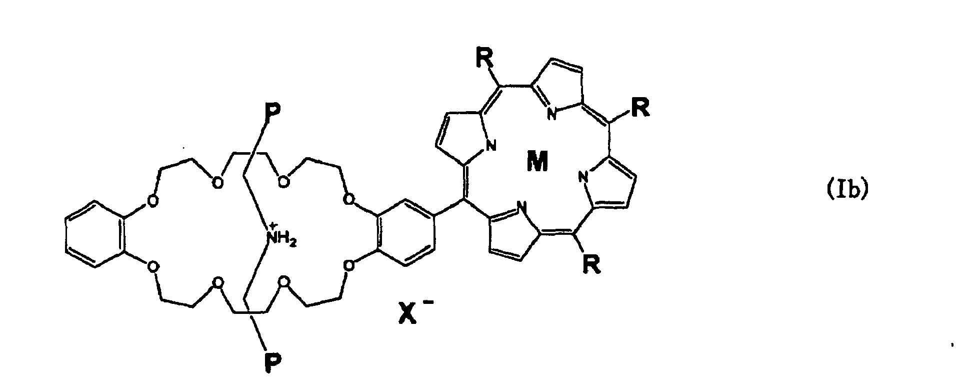 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom, said process comprising reacting a compound represented by the following general formula (IIIb): 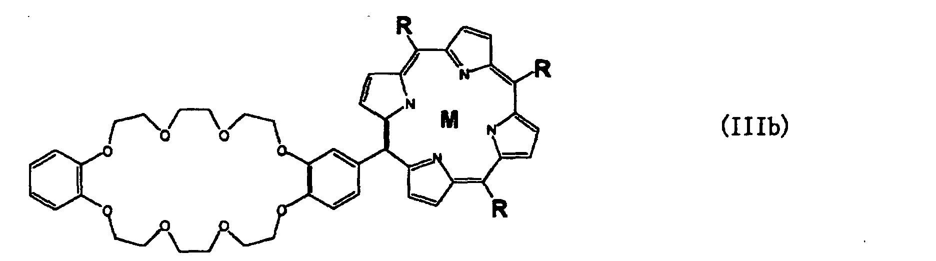 wherein M represents a transition metal coordinatable with the four nitrogen atoms and two additional ligands and R represents a hydrocarbyl group selected from aliphatic hydrocarbon groups and aromatic hydrocarbon groups, with a secondary ammonium salt represented by the following general formula (IVb): 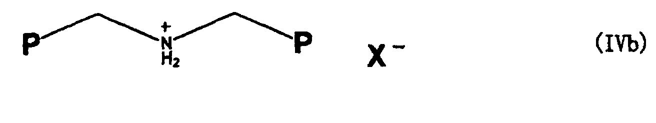 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, and X represents an arbitrary anion atom, in a solvent.
9. A polymer represented by the following general formula (IIa): 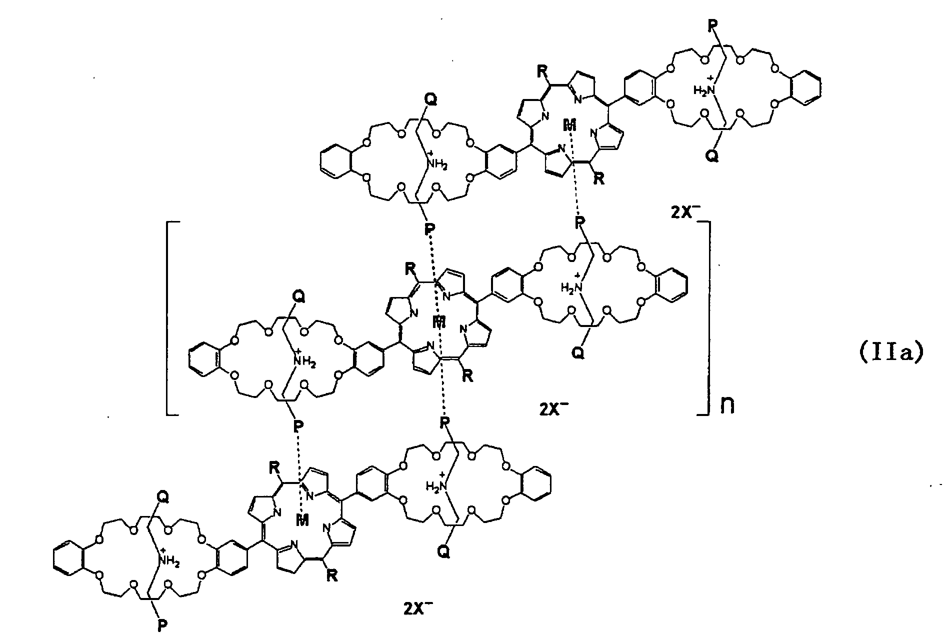 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more.
10. A polymer represented by the following general formula (IIb): 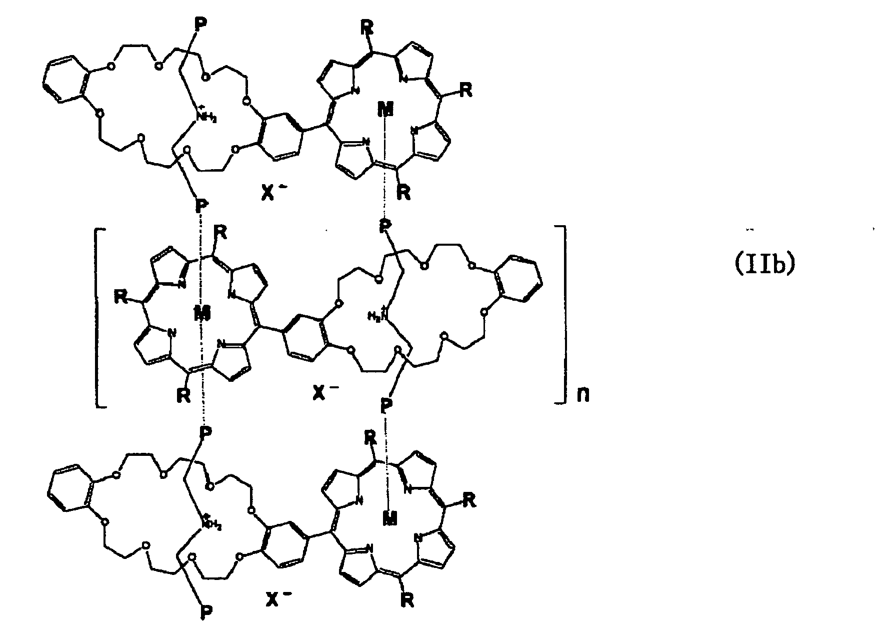 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more.
11. A process for the preparation of a polymer represented by the following general formula (IIa): 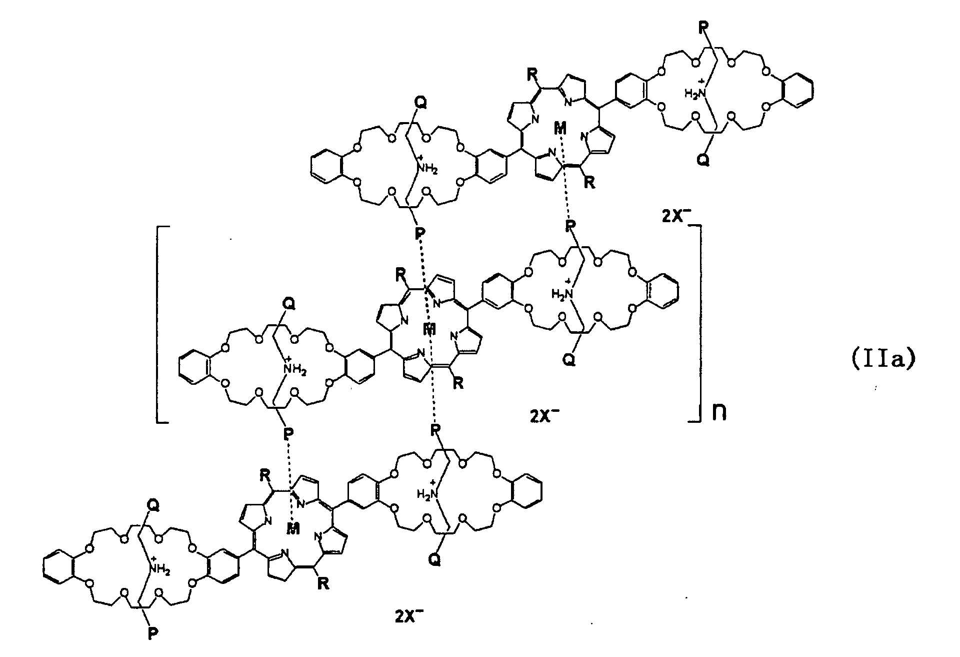 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more,
   said process comprising polymerizing a monomer represented by the following general formula (Ia): 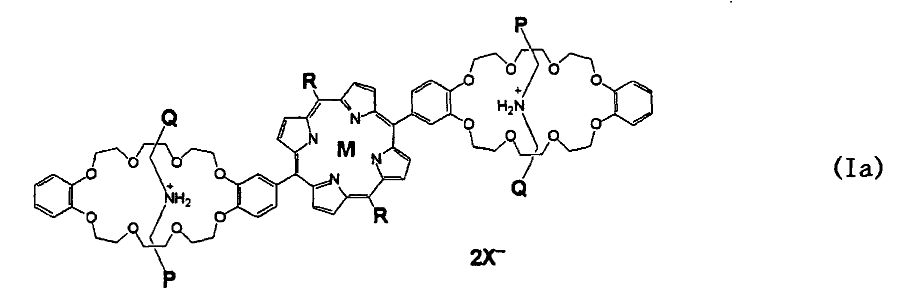 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.
12. A process for the preparation of a polymer represented by the following general formula (IIb): 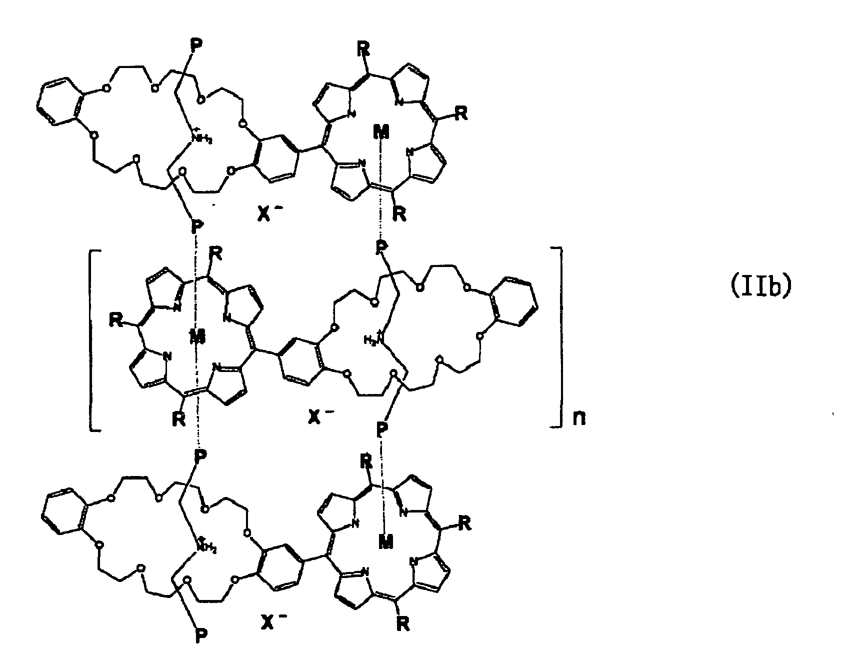
wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more,
said process comprising polymerizing a monomer represented by the following general formula (Ib): 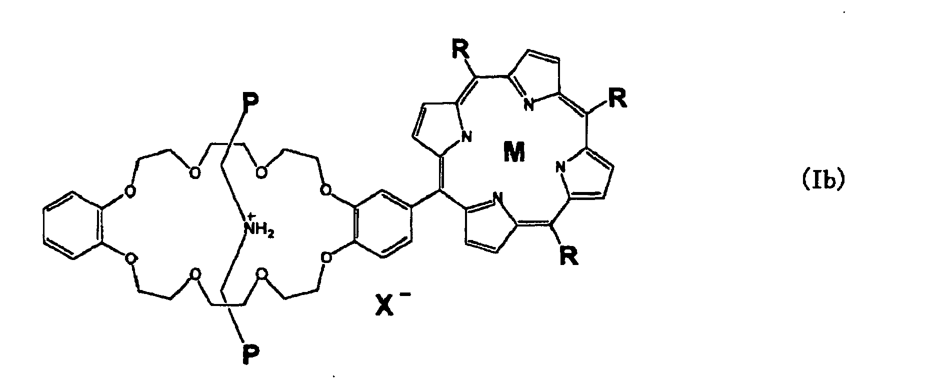 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.

### Best Mode for Carrying Out the Invention:

A first monomer A of the present invention has a chemical structure represented by the following general formula (Ia): 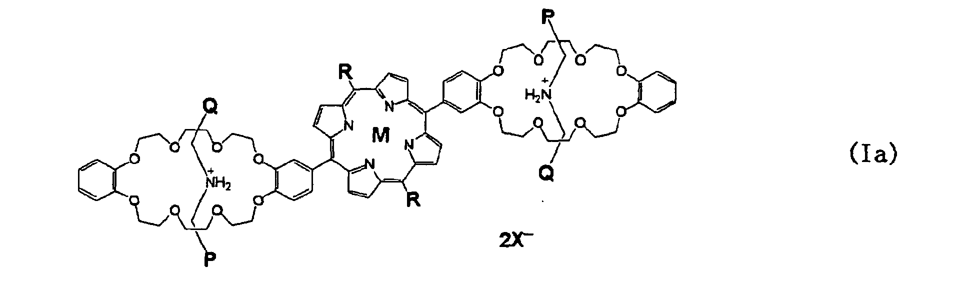 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion.

It is necessary that at least one of P and Q have such a structure that the size thereof is smaller than the inside diameter of the dibenzo-24-crown-8-ether so that it can pass therethrough.

R represents a hydrocarbyl group selected from aliphatic hydrocarbon groups and aromatic hydrocarbon groups. The hydrocarbyl group may have a substituent selected from ethers and esters and has a structure that does not hinder the coordination of the group P with a transition metal.

X represents an arbitrary anion which assures solubility in a non-polar organic solvent.

As long as the above conditions are met, the monomer A can have any substituent.

The monomer A represented by the general formula (Ia) will be further concretely explained.

The transition metal M is a transition metal coordinatable with the four nitrogen atoms located therearound as ligands and with two additional ligands. As the metal, there may be mentioned cobalt, iron, nickel, manganese, rhodium, iridium, gold, silver and platinum.

The group P is a group formed by linking, directly or through a linkage such as an ester linkage, an amide linkage or an ether linkage, a hydrocarbyl group selected from aliphatic hydrocarbon groups and aromatic hydrocarbon groups to a group having at its terminus a nitrogen atom coordinatable with a metal.

The group having a nitrogen atom at a terminus thereof may be an amine which is less basic than a secondary alkylamine and which is coordinatable with a metal. Specific examples of the amine include pyridyl, pyridazinyl, phenylamine, imidazolyl, quinolyl, pyrimidyl, pyrrolidyl, indolyl and indolinyl groups. These groups include their isomers. For example, the pyridyl group may be any of 2-pyridyl, 3-pyridyl and 4-pyridyl groups. The imidazolyl group may be any of 1-imidazolyl, 2-imidazolyl and 4-imidazolyl groups.

The aliphatic hydrocarbon group is a straight chain or a branched chain saturated aliphatic hydrocarbon group having 1-8 carbon atoms. Specific examples of the aliphatic hydrocarbon group include methylene, ethylene, propylene, isopropylene, butylene, isobutylene, pentyl, isopentyl, n-hexene, isohexene, n-heptene, isoheptene, n-octene and isooctene. The aromatic hydrocarbon group may have or may not have a substituent. Specific examples of the aromatic hydrocarbon group include phenyl, phenylene, benzyl, benzylidene, tolyl, xylyl, biphenyl, biphenylene, naphthyl, naphthylene, naphthalenyl and anthracenyl groups. These aromatic hydrocarbon groups may have an aliphatic hydrocarbon group.

The group Q is a group selected from aliphatic hydrocarbon groups and aromatic hydrocarbon groups. These hydrocarbon groups may be linked to other hydrocarbon groups directly or through a linkage such as an ester linkage, an amide linkage or an ether linkage. The aliphatic hydrocarbon group is a straight chain or a branched chain saturated aliphatic hydrocarbon group having 1-8 carbon atoms. Specific examples of the aliphatic hydrocarbon group include methyl, ethyl, propanyl, isopropanyl, n-butyl, i-butyl, isobutyl, tert-butyl, n-pentyl, i-pentyl, tert-pentyl, cyclopentyl, n-hexyl, cyclohexyl, n-heptyl, i-heptyl, tert-heptyl, n-octane, i-octyl and tert-octyl. The aromatic hydrocarbon group may have or may not have a substituent. Specific examples of the aromatic hydrocarbon group include phenyl, phenylene, benzyl, benzylidene, tolyl, xylyl, biphenyl, biphenylene, naphthyl, naphthalenyl, naphthalenyl and anthracenyl groups. The use of the aromatic hydrocarbon group rather than the aliphatic hydrocarbon group is desirable because the resulting secondary ammonium salt can strongly interact with dibenzo-24-crown-8-ether.

One of the groups P and Q must have such a size as to be permitted to pass through the center part of dibenzo-24-crown-8-ether. For example, 3,5-dimethylbenzene and 4-tert-butyl benzene are too large to pass through dibenzo-24-crown-8-ether. Therefore, these groups cannot be used in the groups P and Q at the same time. A smaller sized molecule than these groups should be used. Benzene or toluene can be used.

R is hydrogen or a hydrocarbyl group selected from aliphatic hydrocarbon groups and aromatic hydrocarbon groups. The aliphatic hydrocarbon group is a straight chain or a branched chain saturated aliphatic hydrocarbon group having 1-8 carbon atoms. Specific examples of the aliphatic hydrocarbon group include methyl, ethyl, propanyl, isopropanyl, butyl, isobutyl, tert-butyl, n-butanyl, i-butanyl, tert-butanyl, n-pentyl, i-pentyl, tert-pentyl, n-hexyl, i-hexyl, n-octyl, i-octyl and tert-octyl.

The aromatic hydrocarbon group may have or may not have a substituent. Specific examples of the aromatic hydrocarbon group include phenyl, phenylene, benzyl, benzylidene, tolyl, xylyl, biphenyl, biphenylene, naphthyl, naphthalenyl, anthracenyl, 3,5-di-tert-butylbenzyl, 3,5-dimethoxybenzyl and 3,5-di-oligoethyleneglycolbenzyl groups. In order to effectively obtain intermolecular hydrogen bonding, solubility in a non-polar solvent is an important factor in the above groups. Thus, preference is given to a long chain alkyl group having 6-20 carbon atoms, an ester having a long chain alkyl group, a polyalkyl ether chain or benzene having a tert-butyl group. Specifically, tert-butylbenzene, dimethyl isophthalate and 3,5-dialkoxylbenzene are preferred.

X represents an arbitrary anion. Since the reaction is carried out in a non-polar organic solvent, the anion is preferred to be soluble in the non-polar organic solvent. Specifically, perchlorate ion, hexafluorophosphate ion and trifluoroacetate ion are preferred.

The above-described monomers and polymers obtainable therefrom are novel compounds which are not disclosed in literatures and which are prepared as follows.

First, a compound of the above general formula (IIIa) is prepared in the following manner.

A dipyrrolmethane derivative of the following formula (Va): 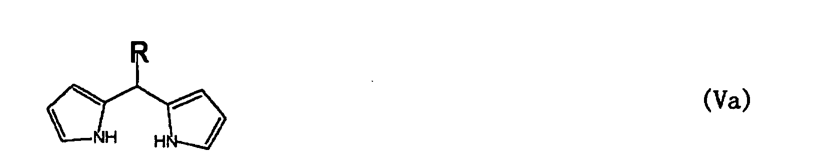 (wherein R is as defined above)
is reacted with an aldehyde-substituted dibenzo-24-crown-8-ether derivative represented by the following general formula (VIa): 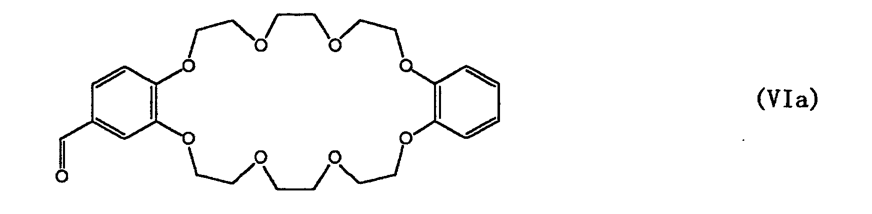 in the presence of an acid catalyst using an oxidizing agent.

As the acid catalyst, there may be generally used, for example, propionic acid or trifluoroacetic acid. As the oxidizing agent, there may be used, for example, 2,3-dichloro-5,6-dicyano-p-benzoquinone or chloranyl.

By the above procedures, 5,15-dibenzo-24-crown-8-ether-disubstituted porphyrin derivative of the following formula (VIIa): 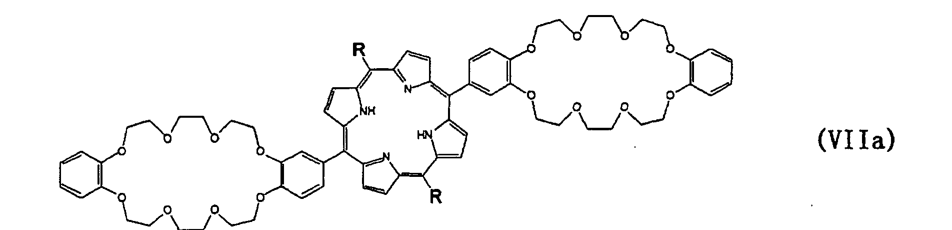 (wherein R is as defined above)
is prepared.

The thus obtained derivative (VIIa) is reacted with an acetic acid salt or chloride of a transition metal M to obtain a transition metal complex of 5,15-dibenzo-24-crown-8-ether-disubstituted porphyrin derivative of the following formula (IIIa): 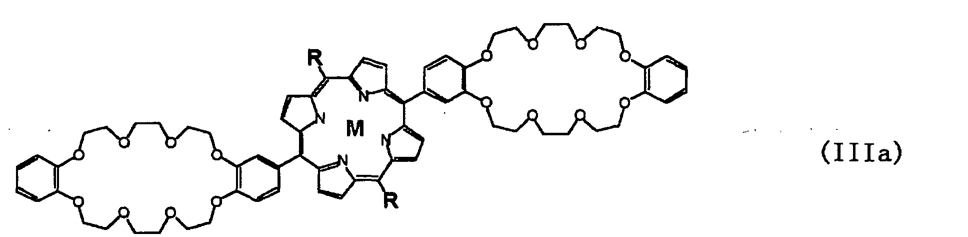 (wherein R and M are as defined above).
The above reaction is carried out in a liquid phase in the presence of a solvent at a temperature of 10-40°C.

Next, the secondary ammonium salt moiety is prepared as follows. An aldehyde derivative of the following general formula (VIIIa): 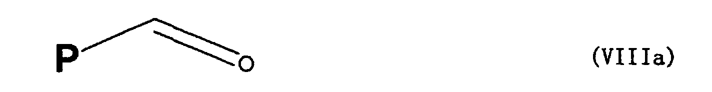 (wherein P is as defined above)
and a primary amine derivative of the following general formula (IXa): 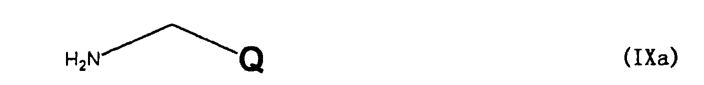 (wherein Q is as defined above)
are heated in a solvent (for example, toluene) to cause dehydration and to obtain a P- and Q-substituted imine derivative of the following general formula (Xa): 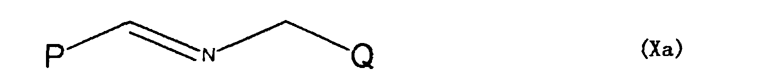 (wherein P and Q are as defined above).
This is then reduced with hydrogenated sodium borate, to which an acid is added to produce the secondary ammonium salt of the following general formula (IVa): 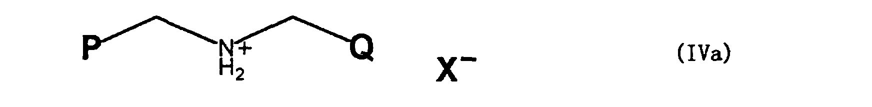 (wherein P and Q are as defined above).

The thus produced transition metal complex of 5,15-dibenzo-24-crown-8-ether-disubstituted porphyrin derivative of the following formula (IIIa): 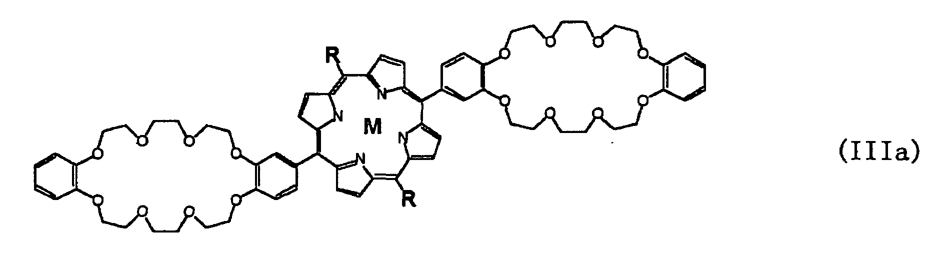 (wherein R and M are as defined above)
and the secondary ammonium salt of the following general formula (IVa): 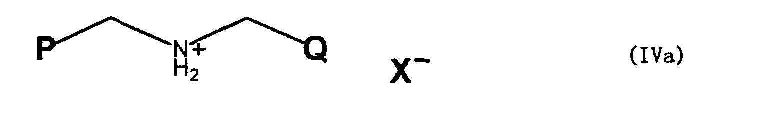 (wherein P and Q are as defined above)
are then reacted in a non-polar solvent. As the non-polar solvent, there may be mentioned methylene chloride or chloroform. When the above compounds are used in an amount of providing a ratio of (IIIa):(IVa) of 1:2, a monomer of the following general formula (Ia): 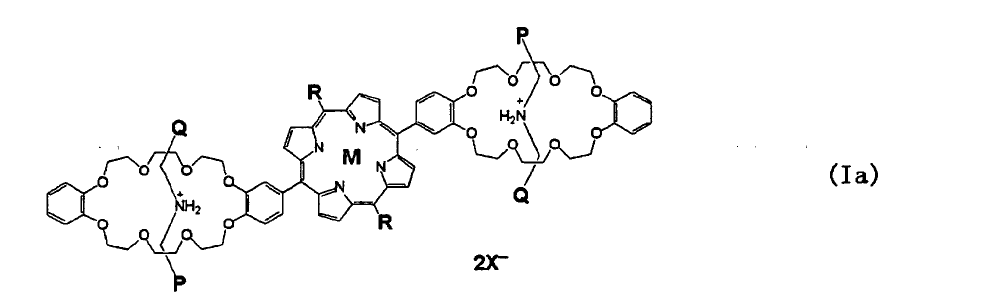 (wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom)
is obtained by reaction at 10-40°C.

Simultaneously, a polymer of the general formula (IIa): 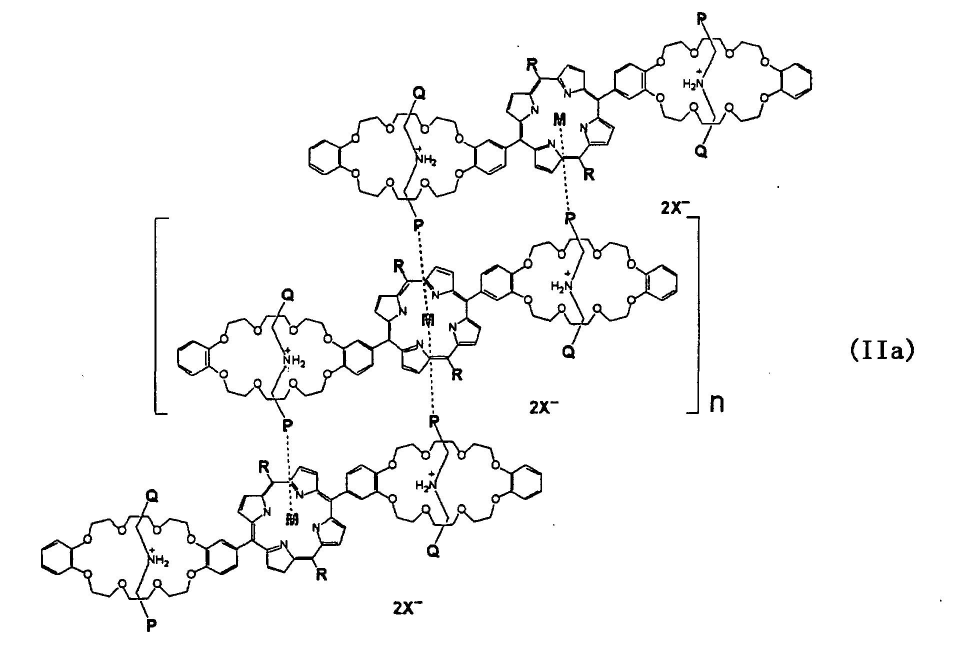 (wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more)
is obtainable by coordination interaction between the nitrogen atoms with the central metal and by hydrogen bonding interaction between the crown ether and the secondary ammonium salt.

The characteristics of the polymer depends upon the number n. The number n is generally an integer of 1 or more. In general, a polymer having n of about 100 is obtained.

The polymer obtained in the present invention has the characteristics of porphyrin having a large π-electron system as will be appreciated from the structure thereof. Because of the characteristics, the polymer has conductivity and can be utilized as a molecular wire. In the case of utilization as a conductive material, the polymer can be easily decomposed to constituent monomer units under mild conditions which inhibit the coordination. with the metal and the hydrogen bonding. Thus, the material can be said to be recyclable.

A second monomer B of the present invention has a chemical structure represented by the following general formula (Ib): 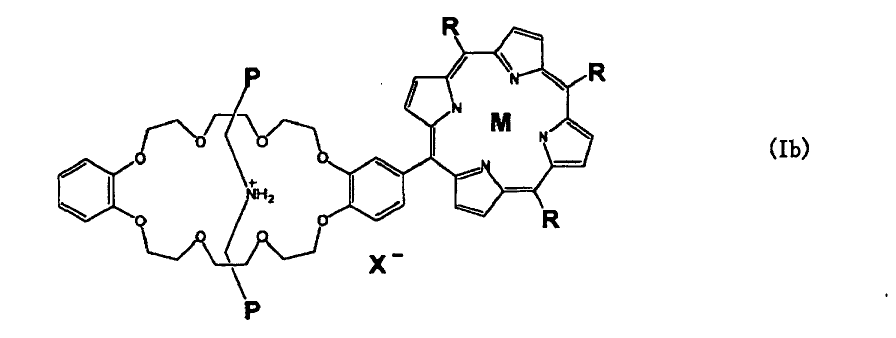 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion.

It is necessary that P have such a structure that the size thereof is smaller than the inside diameter of the dibenzo-24-crown-8-ether so that it can pass therethrough.

R represents a hydrocarbyl group selected from aliphatic hydrocarbon groups and aromatic hydrocarbon groups. The hydrocarbyl group may have a substituent selected from ethers, esters and amides and has a structure that does not hinder the coordination of the group P with a transition metal.

X represents an arbitrary anion which assures solubility in a non-polar organic solvent.

As long as the above conditions are met, the monomer B can have any substituent.

The monomer B represented by the general formula (Ib) will be further concretely explained.

The transition metal M is a transition metal coordinatable with the four nitrogen atoms located therearound as ligands and with two additional ligands. As the metal, there may be mentioned cobalt, iron, nickel, manganese, rhodium, iridium, gold, silver and platinum.

The group P is a group formed by linking, directly or through a linkage such as an ester linkage, an amide linkage or an ether linkage, a hydrocarbyl group selected from aliphatic hydrocarbon groups and aromatic hydrocarbon groups to a group having at its terminus a nitrogen atom coordinatable with a metal.

The group having a nitrogen atom at a terminus thereof may be an amine which is less basic than a secondary alkylamine and which is coordinatable with a metal. Specific examples of the amine include pyridyl, pyridazinyl, phenylamine, imidazolyl, quinolyl, pyrimidyl, pyrrolidyl, indolyl and indolinyl groups. These groups include their isomers. For example, the pyridyl group may be any of 2-pyridyl, 3-pyridyl and 4-pyridyl groups. The imidazolyl group may be any of 1-imidazolyl, 2-imidazolyl and 4-imidazolyl groups.

The aliphatic hydrocarbon group is a straight chain or a branched chain saturated aliphatic hydrocarbon group having 1-8 carbon atoms. Specific examples of the aliphatic hydrocarbon group include methylene, ethylene, propylene, isopropylene, butylene, isobutylene, pentyl, isopentyl, n-hexene, isohexene, n-heptene, isoheptene, n-octene and isooctene. The aromatic hydrocarbon group may have or may not have a substituent. Examples of the aromatic hydrocarbon group include phenyl, phenylene, benzyl, benzylidene, tolyl, xylyl, biphenyl, biphenylene, naphthyl, naphthylene, naphthalenyl and anthracenyl groups. These aromatic hydrocarbon groups may have an aliphatic hydrocarbon group.

The use of the aromatic hydrocarbon group rather than the aliphatic hydrocarbon group is desirable because the resulting secondary ammonium salt can strongly interact with dibenzo-24-crown-8-ether.

The group P must have such a size as to be permitted to pass through the center part of dibenzo-24-crown-8-ether. For example, 3,5-dimethylbenzene and 4-tert-butyl benzene are too large to pass through dibenzo-24-crown-8-ether. Therefore, these groups cannot be used in the group P. A smaller sized molecule than these groups should be used. Benzene or toluene can be used.

R is hydrogen or a hydrocarbyl group selected from aliphatic hydrocarbon groups and aromatic hydrocarbon groups. The aliphatic hydrocarbon group is a straight chain or a branched chain saturated aliphatic hydrocarbon group having 1-8 carbon atoms. Specific examples of the aliphatic hydrocarbon group include methyl, ethyl, propanyl, isopropanyl, butyl, isobutyl, tert-butyl, n-butanyl, i-butanyl, tert-butanyl, n-pentyl, i-pentyl, tert-pentyl, n-hexyl, i-hexyl, n-octyl, i-octyl and tert-octyl.

The aromatic hydrocarbon group may have or may not have a substituent. Specific examples of the aromatic hydrocarbon group include phenyl, phenylene, benzyl, benzylidene, tolyl, xylyl, biphenyl, biphenylene, naphthyl, naphthalenyl, anthracenyl, 3,5-di-tert-butylbenzyl, 3,5-dimethoxybenzyl and 3,5-di-oligoethyleneglycolbenzyl groups. In order to effectively obtain intermolecular hydrogen bonding, solubility in a non-polar solvent is an important factor in the above groups. Thus, preference is given to a long chain alkyl group having 6-20 carbon atoms, an ester having a long chain alkyl group, a polyalkyl ether chain or benzene having a tert-butyl group. Specifically, tert-butylbenzene, dimethyl isophthalate and 3,5-dialkoxylbenzene are preferred.

X represents an arbitrary anion. Since the reaction is carried out in a non-polar organic solvent, the anion is preferred to be soluble in the non-polar organic solvent. Specifically, perchlorate ion, hexafluorophosphate ion and trifluoroacetate ion are preferred.

The above-described monomers and polymers obtainable therefrom are novel compounds which are not disclosed in literatures and which are prepared as follows.

First, a compound of the above general formula (IIIb) is prepared in the following manner.

A dipyrrolmethane derivative of the following formula (Vb): 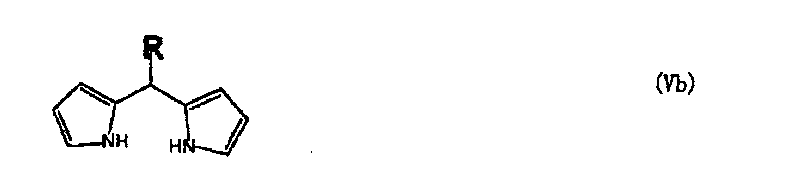 (wherein R is as defined above)
is reacted with an aldehyde-substituted dibenzo-24-crown-8-ether derivative represented by the following general formula (VIb) : 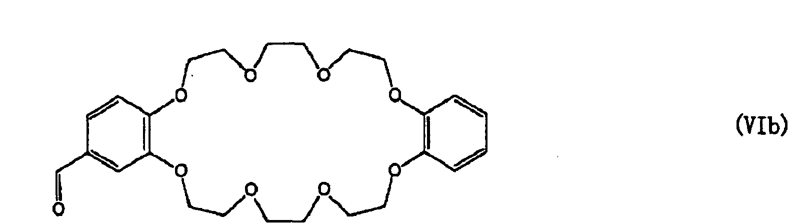 in the presence of an acid catalyst using an oxidizing agent.

As the acid catalyst, there may be generally used, for example, propionic acid or trifluoroacetic acid. As the oxidizing agent, there may be used, for example, 2,3-dichloro-5,6-dicyano-p-benzoquinone or chloranyl.

By the above procedures, 5,15-dibenzo-24-crown-8-ether-substituted porphyrin derivative of the following formula (VIIb): 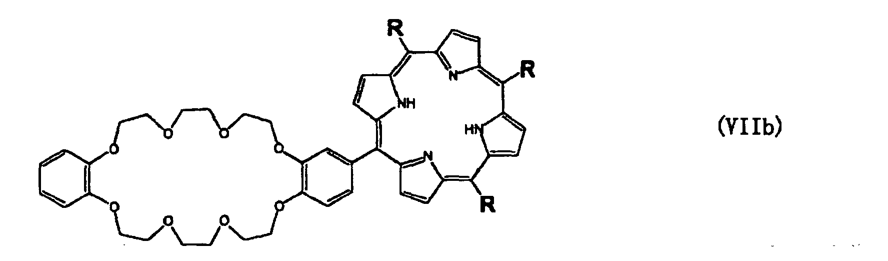 (wherein R is as defined above)
is prepared.

The thus obtained derivative is reacted with an acetic acid salt or chloride of a transition metal M to obtain a transition metal complex of 5,15-dibenzo-24-crown-8-ether-substituted porphyrin derivative of the following formula (IIIb): 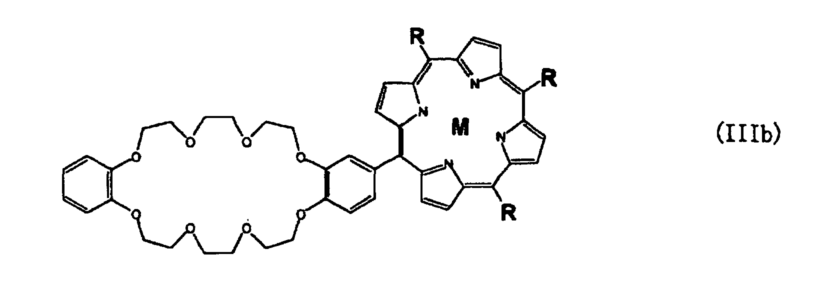 (wherein R and M are as defined above).
The above reaction is carried out in a liquid phase in the presence of a solvent at a temperature of 10-40°C.

Next, the secondary ammonium salt moiety is prepared as follows. An aldehyde derivative of the following general formula (VIIIb): 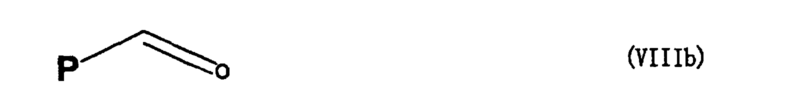 (wherein P is as defined above)
and a primary amine derivative of the following general formula (IXb): 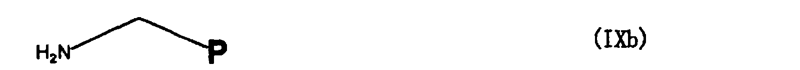 (wherein P is as defined above)
are heated in a solvent (for example, toluene) to cause dehydration and to obtain a P- and Q-substituted imine derivative of the following general formula (Xb): 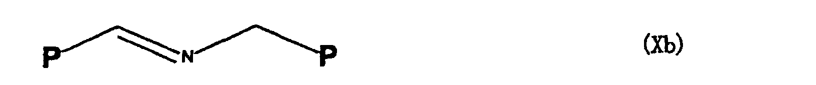 (wherein P and Q are as defined above).
This is then reduced with hydrogenated sodium borate, to which an acid is added to produce the secondary ammonium salt of the following general formula (IVb): 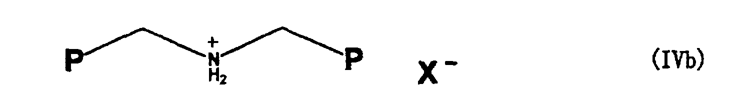 (wherein P is as defined above).

The thus produced transition metal complex of 5,15-dibenzo-24-crown-8-ether-substituted porphyrin derivative of the following formula (IIIb): 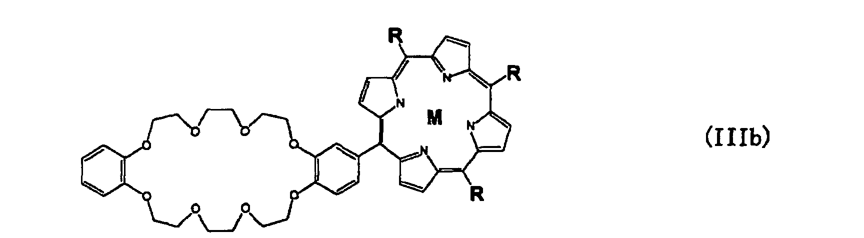 (wherein R and M are as defined above)
and the secondary ammonium salt of the following general formula (IVb): 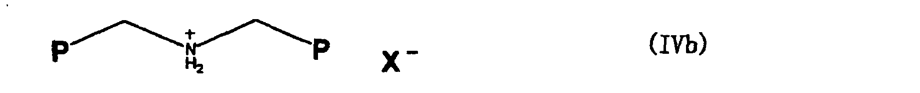 (wherein P is as defined above)
are then reacted in a non-polar solvent. As the non-polar solvent, there may be mentioned methylene chloride or chloroform. When the above compounds are used in an amount of providing a ratio of (IIIb):(IVb) of 1:1, a monomer of the following general formula (Ib): 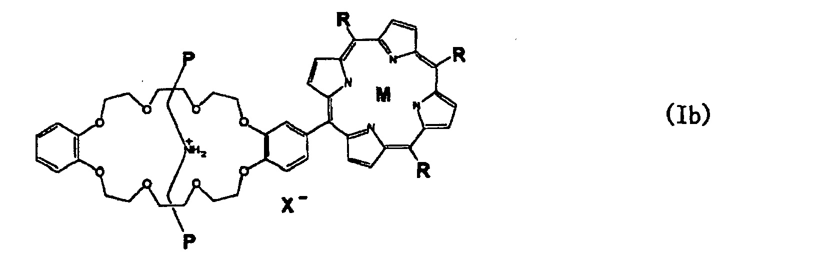 (wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom)
is obtained by reaction at 10-40°C.

Simultaneously, a polymer of the general formula (IIb): 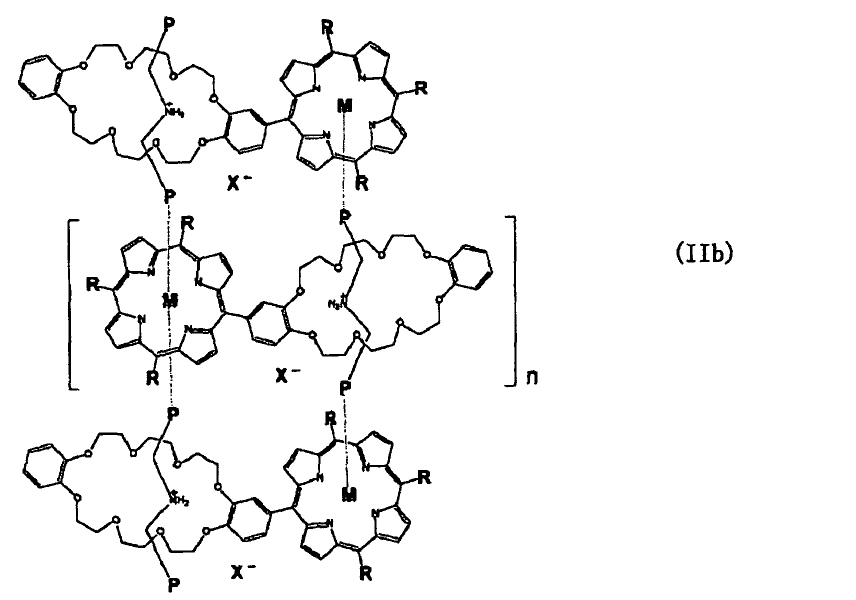 (wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more)
is obtainable by coordination interaction between the nitrogen atoms with the central metal and by hydrogen bonding interaction between the crown ether and the secondary ammonium salt.

The characteristics of the polymer depends upon the number n. The number n is generally an integer of 1 or more. In general, a polymer having n of about 100 is obtained.

The polymer obtained in the present invention has the characteristics of porphyrin having a large π-electron system as will be appreciated from the structure thereof. Because of the characteristics, the polymer has conductivity and can be utilized as a molecular wire. In the case of utilization as a conductive material, the polymer can be easily decomposed to constituent monomer units under mild conditions which inhibit the coordination with the metal and the hydrogen bonding. Thus, the material can be said to be recyclable.

### Examples:

The present invention will be described in more detail by examples. The present invention is not limited to the examples in any way.

### Example 1

In the atmosphere of argon, 0.500 g (2.25 mmol) of 5-phenyldipyromethane and 1.61 g (3.37 mmol) of 6,7,9,10,12,13,20,21,23,24,26,27-dodecahydrodibenzo[b,n],-[1,4,7,10,13,16,18,22]octaoxacyclotetracosin-2-yl aldehyde were dissolved in 300 ml of chloroform, to which 0.125 ml (0.986 mmol) of boron trifluoride diethyl ether complex was added using a syringe while shielding light so that the complex had a concentration of 3.3 mM in the reaction solution. After the reaction mixture had been stirred for 1 hour at room temperature, 0.393 g (1.74 mmol) of 2,3-dichloro-5,6-dicyano-p-benzoquinone was added thereto.
This was stirred for another 1 hour at room temperature. The resulting reaction mixture was washed with an aqueous sodium hydrogen carbonate solution. The crude product was then purified by silica gel chromatography (elution solvent: ethyl acetate) to give 236 mg (16 %) of a porphyrin derivative (XIa) of the following formula (XIa). 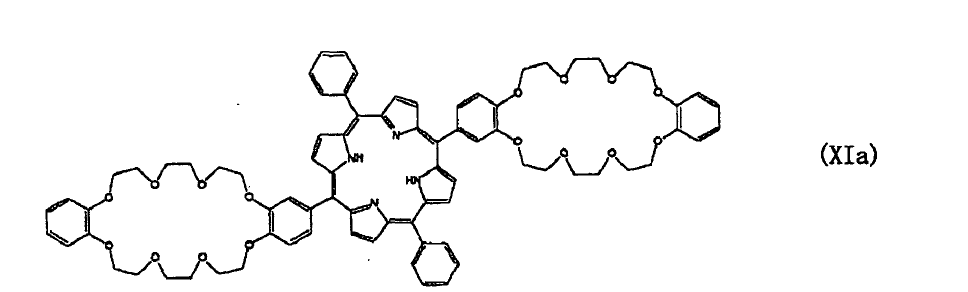 The mass spectrometry gave (as C₈₀H₈₂N₄O₁₆):

| | |
|---|---|
| Calculated value | 1355 |
| Measured value | 1357 (M + 2H) |

### Example 2

The porphyrin derivative of the formula XIa (75 mg; 0.055 mmol) obtained in Example 1 was dissolved in 50 ml of chloroform. While refluxing the chloroform solution, 19 mg (0.076 mmol) of cobalt acetate dissolved in 1 ml of methanol was added thereto. After 1 hour reflux, the reaction mixture was allowed to cool to room temperature and then washed with an aqueous sodium hydrogen carbonate solution. This was then purified by chromatography (silica gel; ethyl acetate). The product was recrystallized from a methylene chloride-methanol mixed solvent to give 37 mg (47 %) of a porphyrin derivative (XIIa) of the following formula (XIIa). 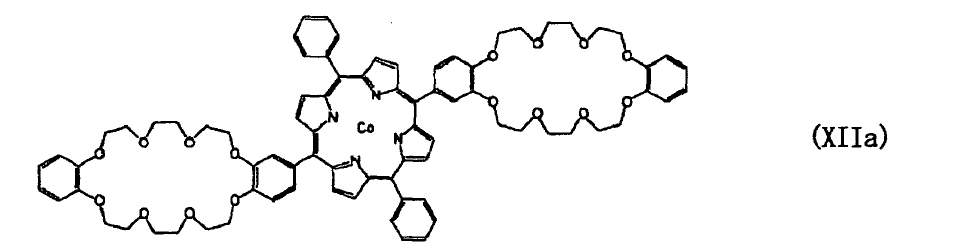 The mass spectrometry gave (as C₈₀H₈₀CoN₄O₁₆):

| | |
|---|---|
| Calculated value | 1412 |
| Measured value | 1435 (M + Na) |

### Example 3

To 1.1 g (6.4 mmol) of methyl 4-formylbenzate and 1.0 g (6.4 mmol) of 4-tert-butylbenzylamine in 100 ml of methylene chloride were added 10 ml of triethylamine and 2.32 g (10 mmol) of anhydrous magnesium sulfate. The mixture was heated under reflux for 10 hours. After separation of solids by filtration, the product was purified by chromatography (silica gel; ethyl acetate) to give 1.6 g (78 %) of an ester derivative (XIIIa) of the following formula (XIIIa). 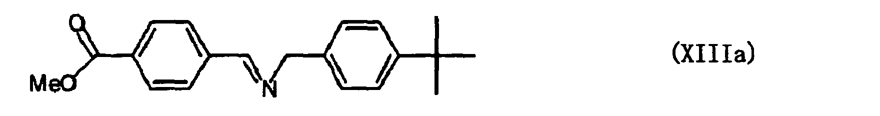 The mass spectrometry gave (as C₂₀H₂₃NO₂):

| | |
|---|---|
| Calculated value | 309 |
| Measured value | 309 |

### Example 4

In 100 ml of methanol were dissolved 1.5 g (4.8 mmol) of the ester derivative (XIIIa) obtained in Example 3 with warming. To this solution, 1 g (26 mmol) of sodium boron hydride and the mixture was stirred for 10 hours at room temperature. The resulting reaction solution was mixed with 50 ml of 2M aqueous hydrochloric acid solution. The product was extracted with 100 ml of chloroform and the organic phase was dried with magnesium sulfate. The solvent was then removed by distillation to give 1.4 g (90 %) of an ester derivative (XIVa) of the following formula (XIVa). 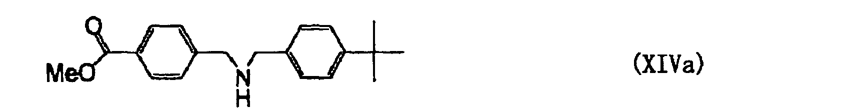 The mass spectrometry gave (as C₂₀H₂₅NO₂):

| | |
|---|---|
| Calculated value | 311 |
| Measured value | 311 |

### Example 5

In 50 ml of chloroform were dissolved 1.2 g (3.9 mmol) of the ester derivative (XIVa), to which 0.85 g (3.9 mmol) of di-tert-butylcarbonate and 5 mg (0.4 mmol) of dimethylaminopyridine were added. After stirring for 5 hours, the reaction solution was washed with 50 ml of an aqueous 2M hydrochloric acid and 50 ml of distilled water. The organic layer was dried with magnesium sulfate. The solvent was then removed by distillation. The oily substance thus obtained was purified by chromatography (silica gel; ethyl acetate) to give 1.5 g (93 %) of an ester derivative (XVa) of the following formula (XVa). 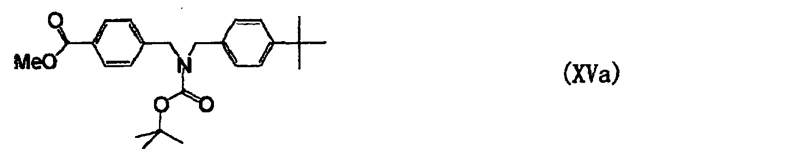 The mass spectrometry gave (as C₂₅H₃₃NO₄):

| | |
|---|---|
| Calculated value | 411 |
| Measured value | 411 |

### Example 6

In 50 ml of methanol were dissolved 1.2 g (2.9 mmol) of the diester derivative (XVa), to which 50 ml of 1M aqueous sodium hydroxide solution were added. After stirring for 3 hours with heating, a half volume of the solvent was removed by distillation. To the resulting mixture, hydrochloric acid was added to adjust the pH to 2. White precipitates were separated by filtration to give 1.0 g (90 %) of a dicarboxylic acid derivative (XVIa) of the following formula (XVIa). 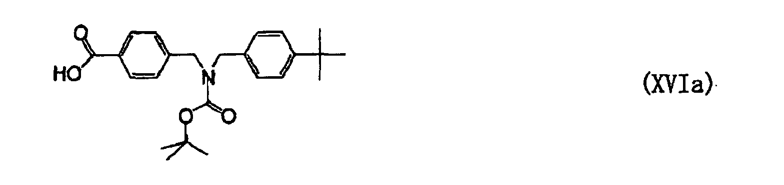 The mass spectrometry gave (as C₂₄H₃₁NO₄):

| | |
|---|---|
| Calculated value | 397 |
| Measured value | 397 |

### Example 7

In 50 ml of dimethylformamide were dissolved 1.0 g (2.5 mmol) of the carboxylic acid derivative (XVIa) obtained in Example 6 and 270 mg (2.8 mmol) of 4-aminopyridine, to which 490 mg (3.0 mmol) of 3-hydroxy-4-oxo-3,4-dihydro-1,2,3-benzotriazine were added. The solution was cooled to 20°C, to which 0.55 ml (3.0 mmol) of N-ethyl-N'-3-dimethylaminopropyl-carbodiimide was added with stirring. The reaction mixture was stirred at room temperature for 6 hours. Then, an excess amount of water was added to the reaction mixture to precipitate the product. The precipitates were filtered and dried to give 1.0 g (84 %) of an amide derivative (XVIIa) of the following formula (XVIIa). 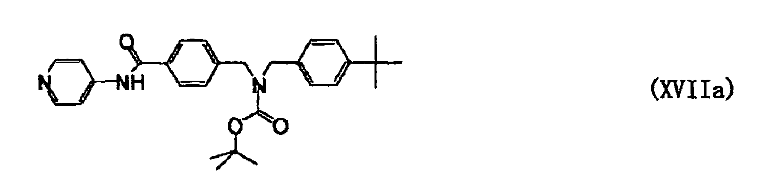 The mass spectrometry gave (as C₂₉H₃₅N₃O₃):

| | |
|---|---|
| Calculated value | 473 |
| Measured value | 473 |

### Example 8

To 1.0 g (2.1 mmol) of the amide derivative (XVIIa) obtained in Example 7 was added 10 ml of trifluoroacetic acid. The mixture was stirred at room temperature for 1 hour. Then trifluoroacetic acid was distilled in vacuo to obtain 980 mg (95 %) of a secondary amine derivative
(XVIIIa) having a pyridyl group and represented by the following formula (XVIIIa): 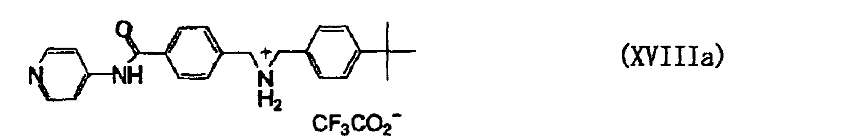 The mass spectrometry gave (as C₂₆H₂₇F₃N₃O₃):

| | |
|---|---|
| Calculated value | 486 |
| Measured value | 486 |

### Example 9

Five (5) mg (3.5 µmol) of the porphyrin derivative (XIIa) obtained in Example 2 and 4.4 mg (7.0 µmol) of the pyridyl group-bearing secondary amine derivative (XVIIIa) obtained in Example 8 were dissolved in chloroform to produce a monomer (XXa) having the following structural formula (XXa):

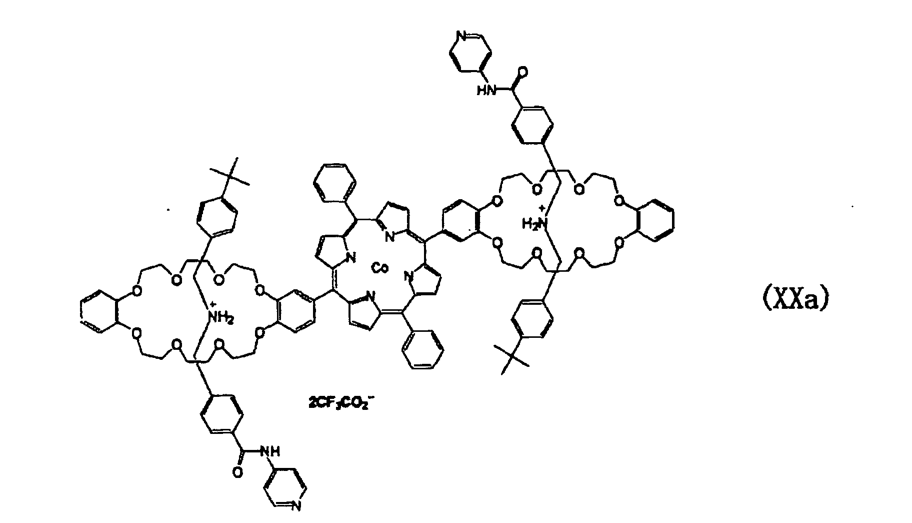

At the same time, the formation of a polymer (XXIa) utilizing the metal coordination interaction and the hydrogen bonding interaction and having the following structural formula (XXIa) was confirmed. 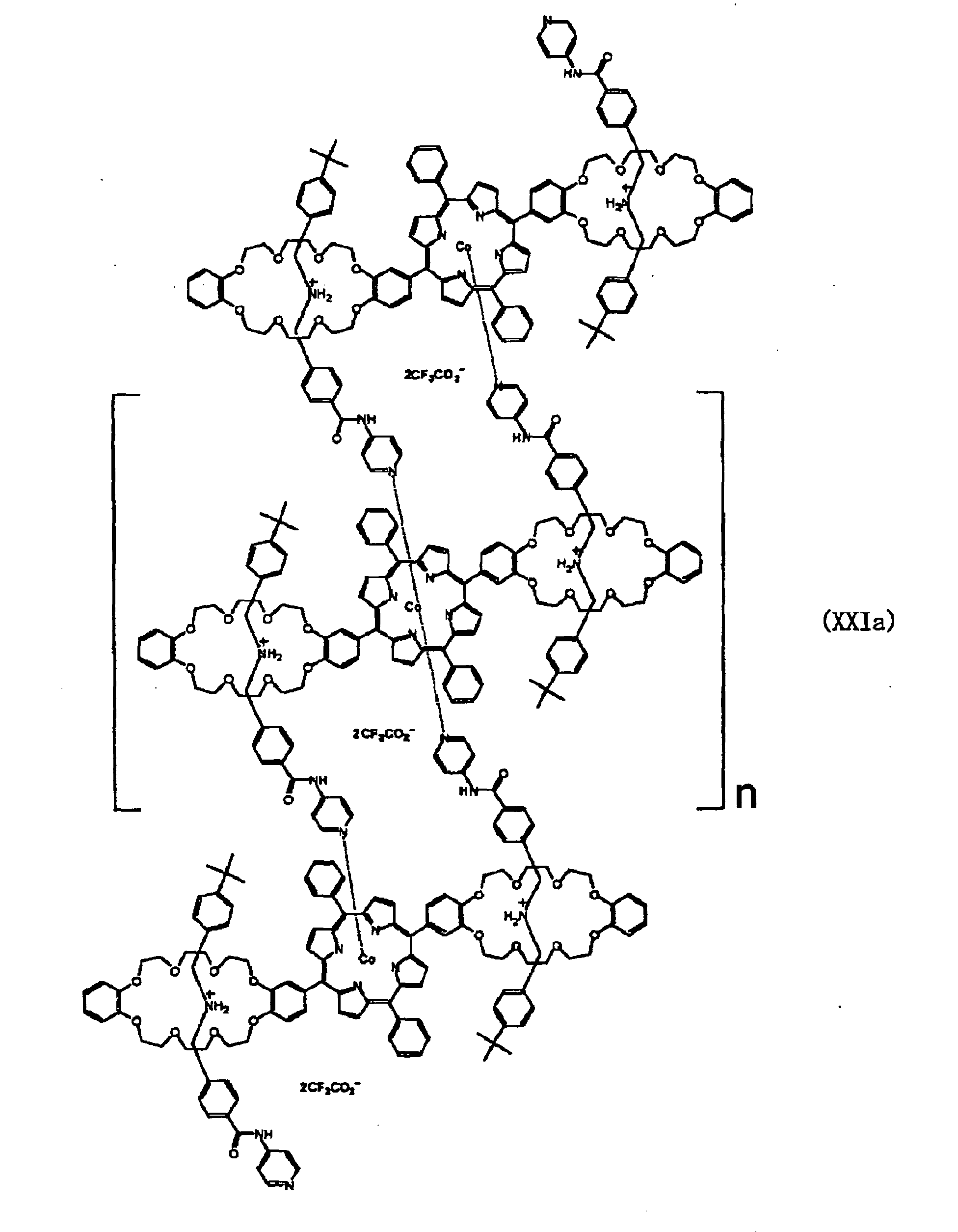 The number n was 100. The mass spectrometry gave (as C₁₃₂H₁₃₆CoF₅N₆O₂₂):

| | |
|---|---|
| Calculated value | 2387 |
| Measured value | 2387, 4774, 7161, 9548, 11935 |

### Example 10

In the atmosphere of argon, 0.328 g (1.48 mmol) of 5-phenyldipyromethane and 0.703 g (1.48 mmol) of 6,7,9,10,12,13,20,21,23,24,26,27-dodecahydrodibenzo[b,n],-[1,4,7,10,13,16,18,22]octaoxacyclotetracosin-2-yl aldehyde were dissolved in 150 ml of chloroform, to which 0.6 ml (7.79 mmol) of trifluoroacetic acid was added using a syringe while shielding light. After the reaction mixture had been stirred for 1 hour at room temperature, 0.285 g (1.14 mmol) of chloranyl dissolved in 0.9 ml of tetrahydrofuran was added to the reaction mixture using a syringe. The reaction mixture was stirred for 5 hours at room temperature. The resulting reaction mixture was washed with an aqueous sodium hydrogen carbonate solution. The crude product was then purified by silica gel chromatography (elution solvent: ethyl acetate) to give 103 mg (14 %) of a porphyrin derivative (XIb) of the following formula (XIb). 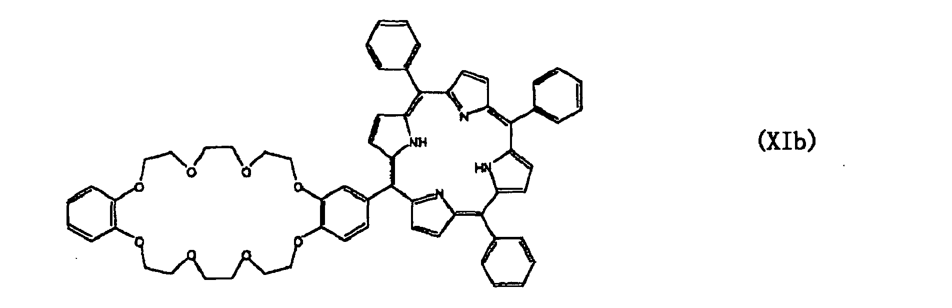 The mass spectrometry gave (as C₆₂H₅₆N₄O₈):

| | |
|---|---|
| Calculated value | 985 |
| Measured value | 987 (M + 2H) |

### Example 11

The porphyrin derivative of the formula (XIb) (66 mg; 0.067 mmol) obtained in Example 10 was dissolved in 50 ml of chloroform. While refluxing the chloroform solution, 22 mg (0.084 mmol) of cobalt acetate dissolved in 1 ml of methanol was added thereto. After 1 hour reflux, the reaction mixture was allowed to cool to room temperature and then washed with an aqueous sodium hydrogen carbonate solution. This was then purified by chromatography (silica gel; ethyl acetate). The product was recrystallized from a methylene chloride-methanol mixed solvent to give 28 mg (40 %) of a porphyrin derivative (XIIb) of the following formula (XIIb). 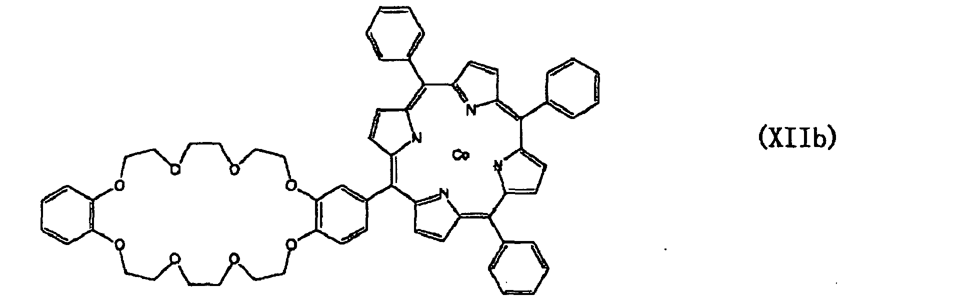 The mass spectrometry gave (as C₈₀H₈₂CoN₄O₁₆):

| | |
|---|---|
| Calculated value | 1042 |
| Measured value | 1042 |

### Example 12

To 1.1 g (6.4 mmol) of methyl 4-formylbenzate and 1.3 g (6.4 mmol) of methyl 4-aminomethyl benzoate in 100 ml of methylene chloride were added 10 ml of triethylamine and 2.32 g (10 mmol) of anhydrous magnesium sulfate. The mixture was heated under reflux for 10 hours. After separation of solids by filtration, the product was purified by chromatography (silica gel; ethyl acetate) to give 1.7 g (80 %) of an ester derivative (XIIIb) of the following formula (XIIIb). 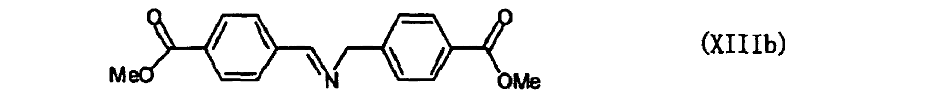 The mass spectrometry gave (as C₁₈H₁₇NO₄):

| | |
|---|---|
| Calculated value | 311 |
| Measured value | 311 |

### Example 13

In 100 ml of methanol were dissolved 1.5 g (4.8 mmol) of the ester derivative (XIIIb) obtained in Example 12 with warming. To this solution, 1 g (26 mmol) of sodium boron hydride and the mixture was stirred for 10 hours at room temperature. The resulting reaction solution was mixed with 50 ml of 2M aqueous hydrochloric acid solution. The product was extracted with 100 ml of chloroform and the organic phase was dried with magnesium sulfate. The solvent was then removed by distillation to give 1.4 g (90 %) of a diester derivative (XIVb) of the following formula (XIVb). 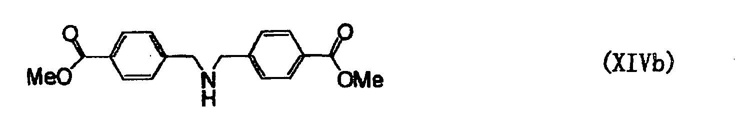 The mass spectrometry gave (as C₁₈H₁₉NO₄):

| | |
|---|---|
| Calculated value | 313 |
| Measured value | 313 |

### Example 14

In 50 ml of chloroform were dissolved 1.2 g (3.8 mmol) of the diester derivative (XIVb) obtained in Example 13, to which 0.83 g (3.8 mmol) of di-tert-butylcarbonate and 5 mg (0.4 mmol) of dimethylaminopyridine were added. After stirring for 5 hours, the reaction solution was washed with 50 ml of an aqueous 2M hydrochloric acid and 50 ml of distilled water. The organic layer was dried with magnesium sulfate. The solvent was then removed by distillation. The oily substance thus obtained was purified by chromatography (silica gel; ethyl acetate) to give 1.4 g (90 %) of a diester derivative (XVb) of the following formula (XVb). 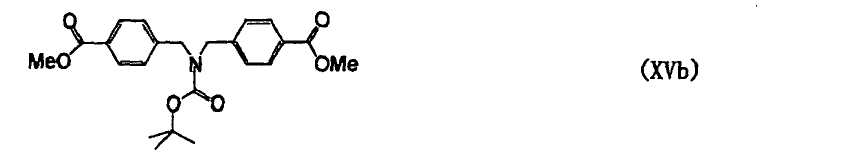 The mass spectrometry gave (as C₂₃H₂₇NO₆):

| | |
|---|---|
| Calculated value | 413 |
| Measured value | 413 |

### Example 16

In 50 ml of methanol were dissolved 1.2 g (2.9 mmol) of the diester derivative (XVb), to which 50 ml of 1M aqueous sodium hydroxide solution were added. After stirring for 3 hours with heating, a half volume of the solvent was removed by distillation. To the resulting mixture, hydrochloric acid was added to adjust the pH to 2. White precipitates were separated by filtration to give 1.1 g (95 %) of a dicarboxylic acid derivative (XVIb) of the following formula (XVIb). 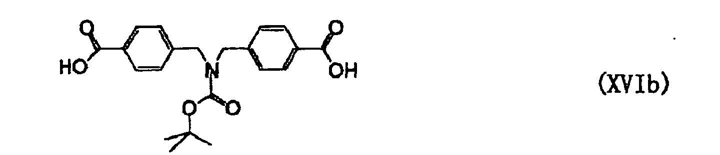 The mass spectrometry gave (as C₂₁H₂₃NO₆):

| | |
|---|---|
| Calculated value | 385 |
| Measured value | 385 |

### Example 16

In 50 ml of dimethylformamide were dissolved 1.0 g (2.6 mmol) of the dicarboxylic acid derivative (XVIb) obtained in Example 15 and 560 mg (5.8 mmol) of 4-aminopyridine, to which 980 mg (6.0 mmol) of 3-hydroxy-4-oxo-3,4-dihydro-1,2,3-benzotriazine were added. The solution was cooled to 20°C, to which 0.55 ml (3.0 mmol) of N-ethyl-N'-3-dimethylaminopropyl-carbodiimide was added with stirring. The reaction mixture was stirred at room temperature for 6 hours. Then, an excess amount of water was added to the reaction mixture to precipitate the product. The precipitates were filtered and dried to give 1.3 g (93 %) of a diamide derivative (XVIIb) of the following formula (XVIIb). 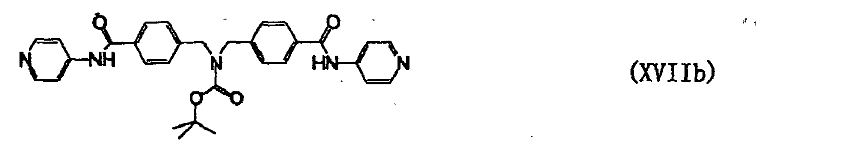 The mass spectrometry gave (as C₃₁H₃₁N₅O₄):

| | |
|---|---|
| Calculated value | 537 |
| Measured value | 537 |

### Example 17

To 1.0 g (1.9 mmol) of the diamide derivative (XVIIb) obtained in Example 16 was added 10 ml of trifluoroacetic acid. The mixture was stirred at room temperature for 1 hour. Then trifluoroacetic acid was distilled in vacuo to obtain 940 mg (92 %) of a secondary amine derivative (XVIIIb) having pyridyl groups at both termini and represented by the following formula (XVIIIb): 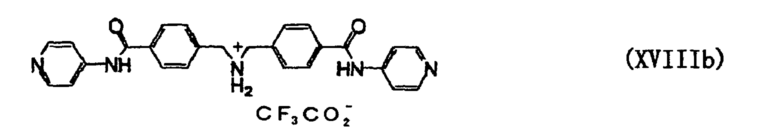 The mass spectrometry gave (as C₂₈H₂₃F₃N₅O₄):

| | |
|---|---|
| Calculated value | 550 |
| Measured value | 550 |

### Example 18

Five (5) mg (4.8 µmol) of the porphyrin derivative (XIIb) obtained in Example 11 and 2.6 mg (4.8 µmol) of the two pyridyl group-terminated secondary amine derivative (XVIIIb) obtained in Example 17 were dissolved in chloroform to produce a monomer (XXb) having the following structural formula (XXb):

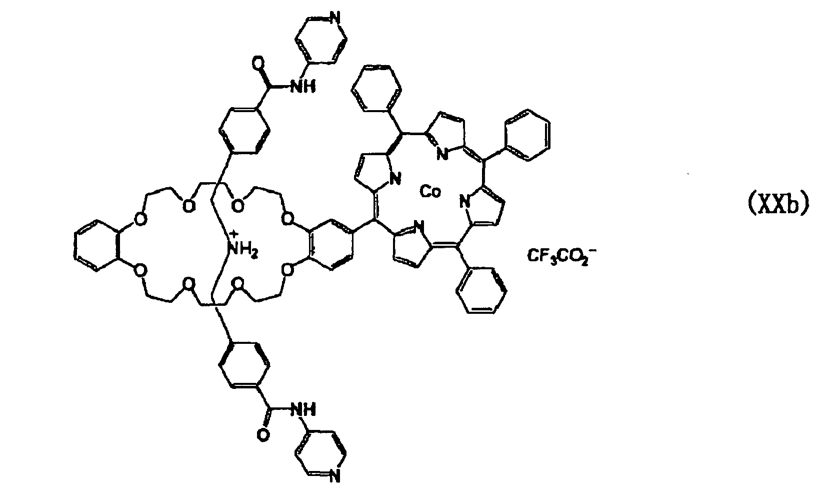

At the same time, the formation of a polymer (XXIb) utilizing the metal coordination interaction and the hydrogen bonding interaction and having the following structural formula (XXIb) was confirmed. 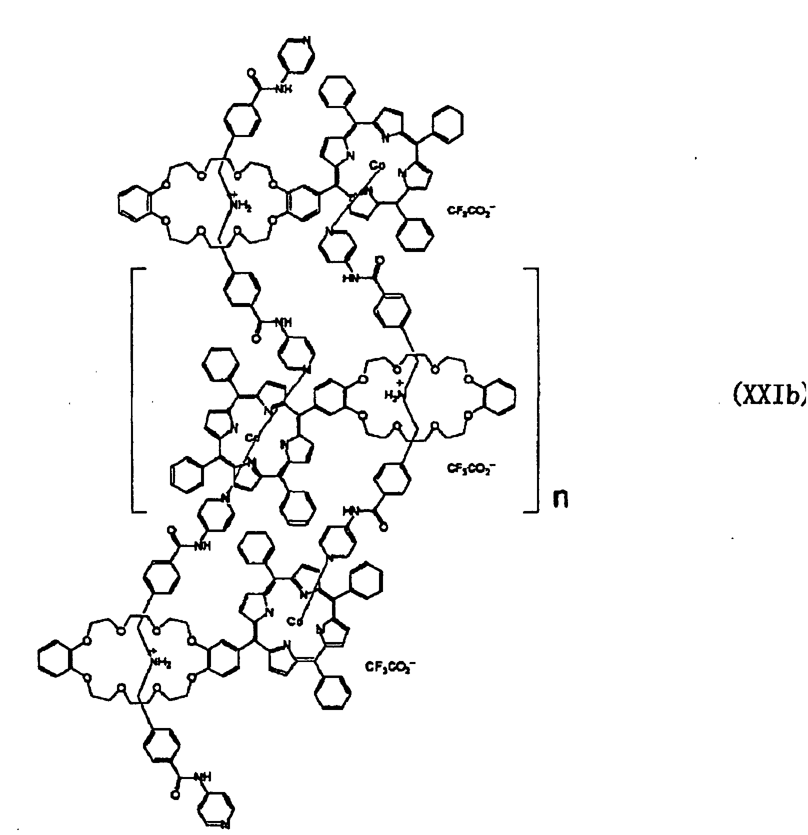 The number n was 100. The mass spectrometry gave (as C₉₀H₇₈CoF₃N₉O₁₂):

| | |
|---|---|
| Calculated value | 1593 |
| Measured value | 1593, 3186, 4779, 6372, 7965 |

### Industrial Applicability:

By using a technique according to the present invention based on a new concept of an organic compound which has not been conventionally known, it is possible to prepare a monomer capable of forming a polymer utilizing metal coordination interaction and hydrogen bonding interaction. It is possible to obtain a polymer by polymerization of the monomer through spontaneous assembly thereof. The polymer of the present invention can be easily decomposed by controlling the external environment and, therefore, can be utilized as a recyclable clean polymer. By utilization of the conjugated π-electron of the porphyrin rings, the polymer is expected to be utilized as a molecular wire material which provides a basis for molecular elements which are constituent parts of a molecular computer.

## Claims

1. A monomer represented by the following general formula (Ia): 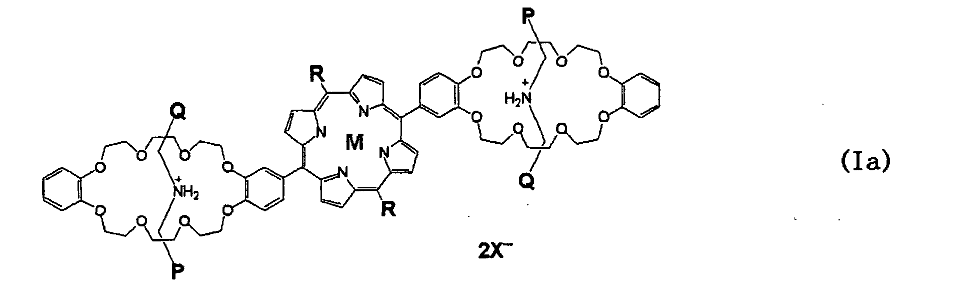 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.

2. A monomer represented by the following general formula (Ib) : 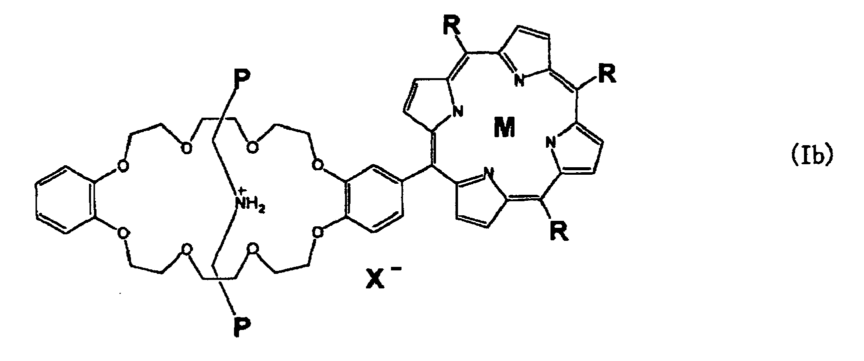 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.

3. A compound represented by the following general formula (IIIa): 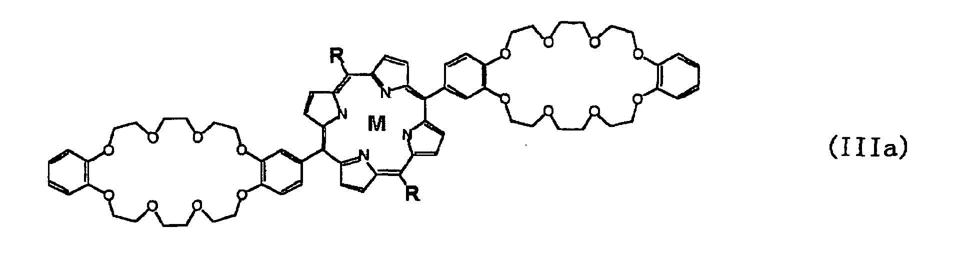 wherein M represents a transition metal coordinatable with the four nitrogen atoms and two additional ligands and R represents a hydrocarbyl group selected from aliphatic hydrocarbon groups and aromatic hydrocarbon groups.

4. A compound represented by the following general formula (IIIb): 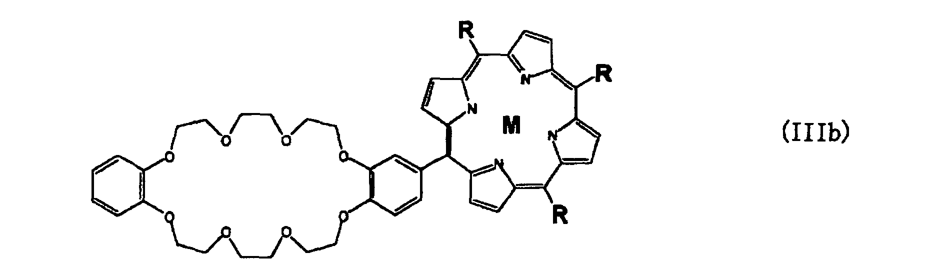 wherein M represents a transition metal coordinatable with the four nitrogen atoms and two additional ligands and R represents a hydrocarbyl group selected from aliphatic hydrocarbon groups and aromatic hydrocarbon groups.

5. A secondary ammonium salt represented by the following general formula (IVa): 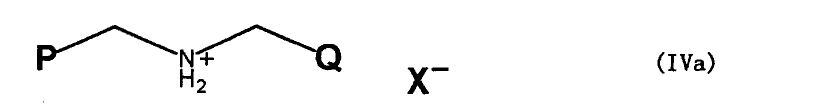 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups and X represents an arbitrary anion atom.

6. A secondary ammonium salt represented by the following general formula (IVb): 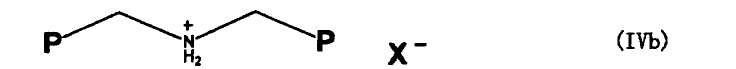 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, and X represents an arbitrary anion atom.

7. A process for the preparation of a monomer represented by the following general formula (Ia): 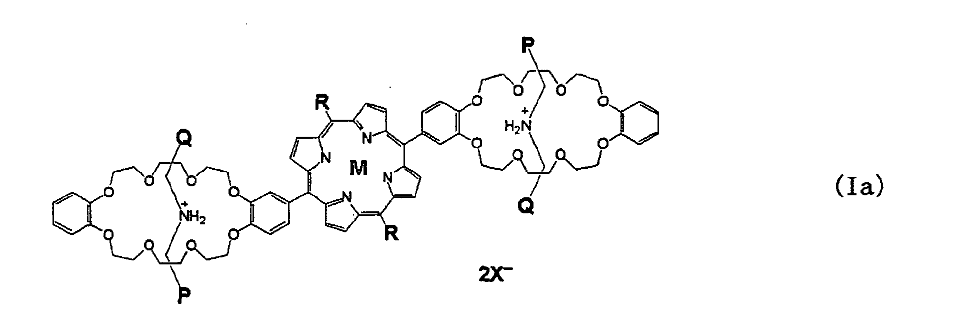 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom, said process comprising reacting a compound represented by the following general formula (IIIa): 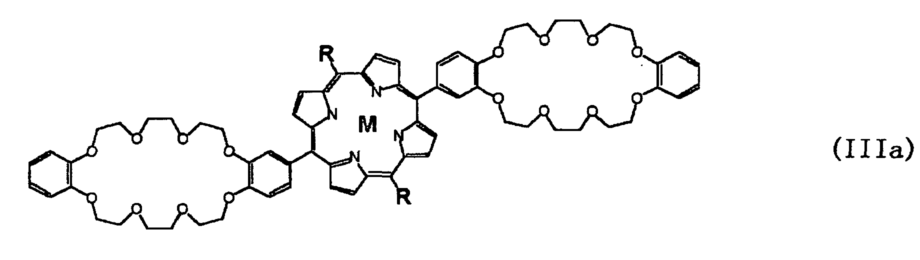 wherein M represents a transition metal coordinatable with the four nitrogen atoms and two additional ligands and R represents a hydrocarbyl group selected from aliphatic hydrocarbon groups and aromatic hydrocarbon groups, with a secondary ammonium salt represented by the following general formula (IVa): 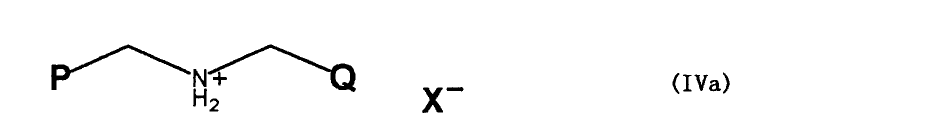 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups and X represents an arbitrary anion atom, in a solvent.

8. A process for the preparation of a monomer represented by the following general formula (Ib): 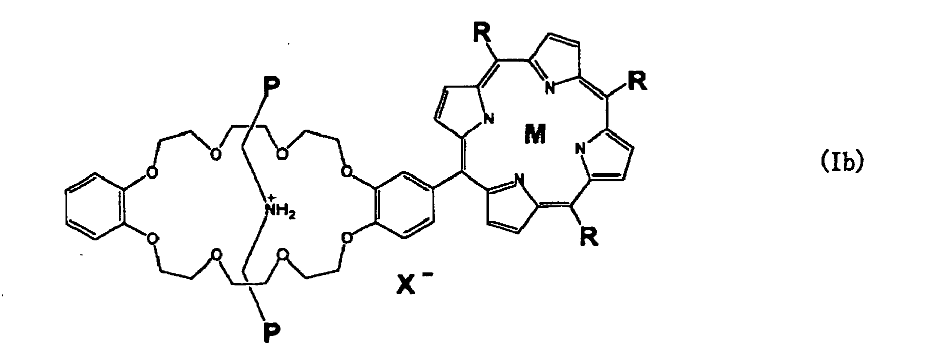 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom, said process comprising reacting a compound represented by the following general formula (IIIb): 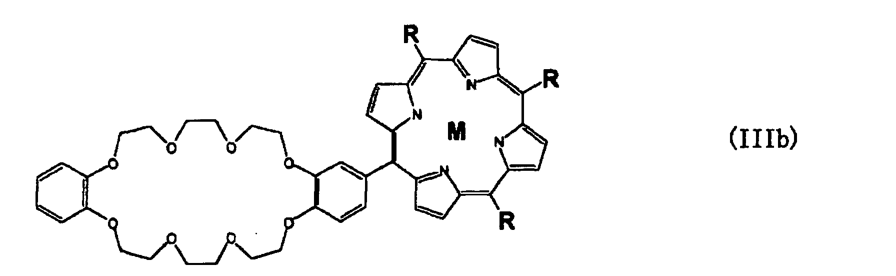 wherein M represents a transition metal coordinatable with the four nitrogen atoms and two additional ligands and R represents a hydrocarbyl group selected from aliphatic hydrocarbon groups and aromatic hydrocarbon groups, with a secondary ammonium salt represented by the following general formula (IVb): 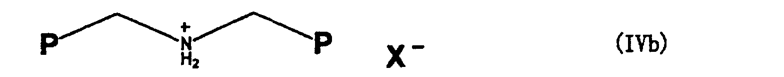 wherein P represents a group having, at a terminus thereof through a hydrocarbyl group, a nitrogen atom coordinatable with a metal, said hydrocarbyl group being selected from aliphatic hydrocarbon groups and aromatic hydrocarbon groups, and X represents an arbitrary anion atom, in a solvent.

9. A polymer represented by the following general formula (IIa): 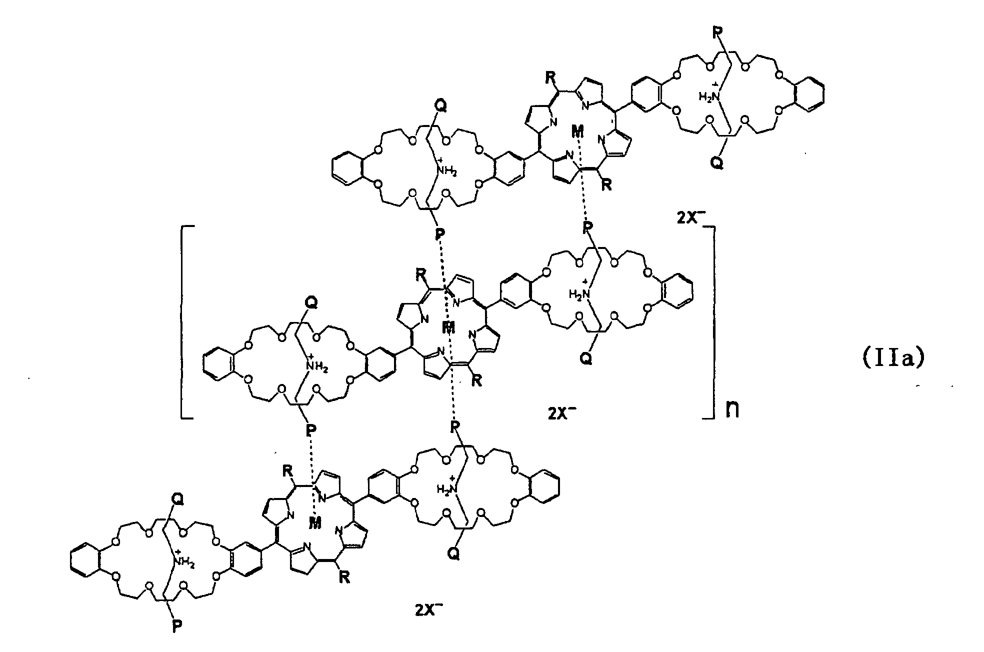 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more.

10. A polymer represented by the following general formula (IIb): 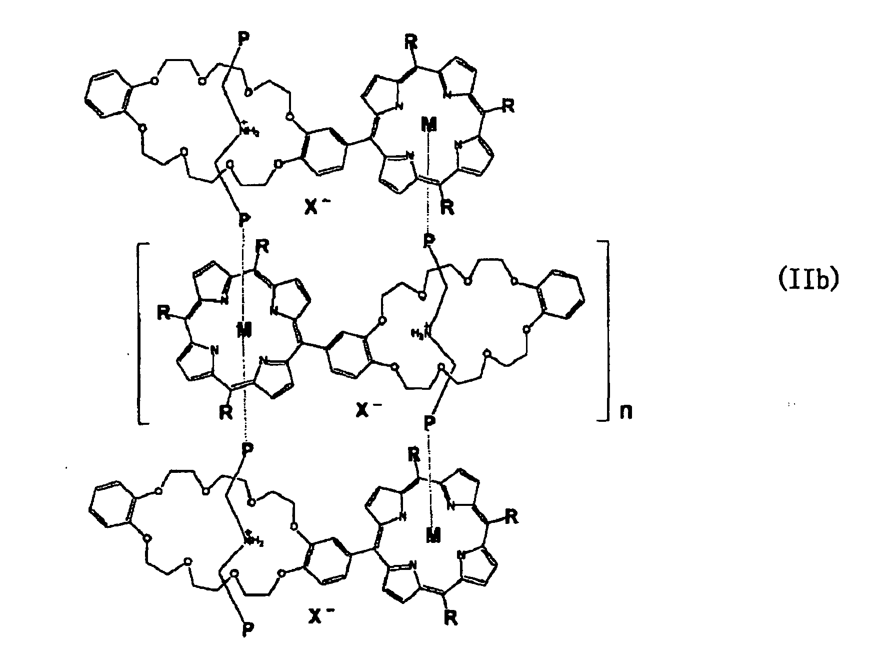 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more.

11. A process for the preparation of a polymer represented by the following general formula (IIa): 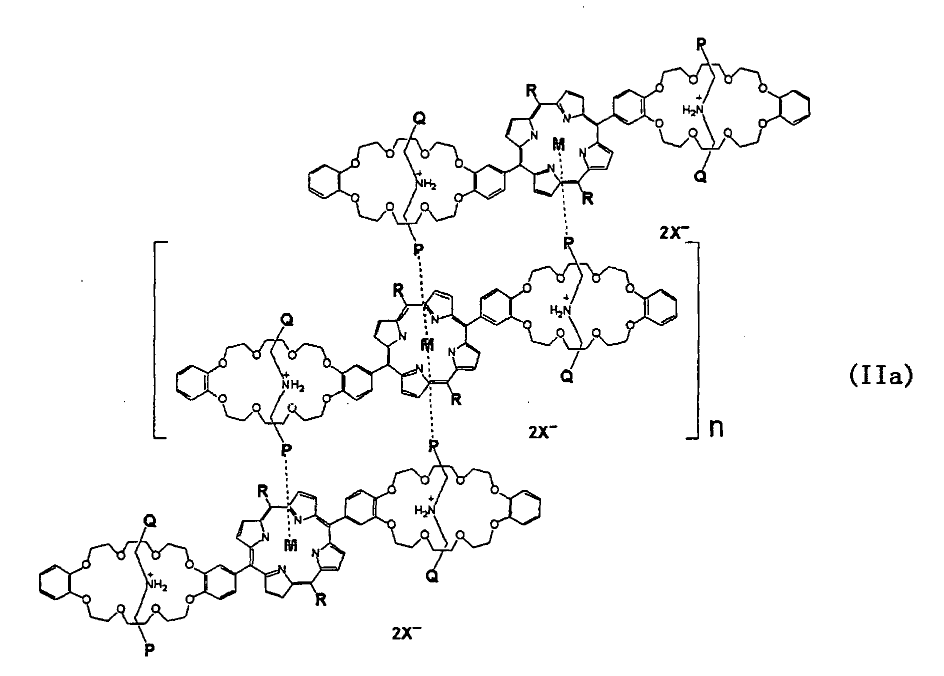 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more,
said process comprising polymerizing a monomer represented by the following general formula (Ia): 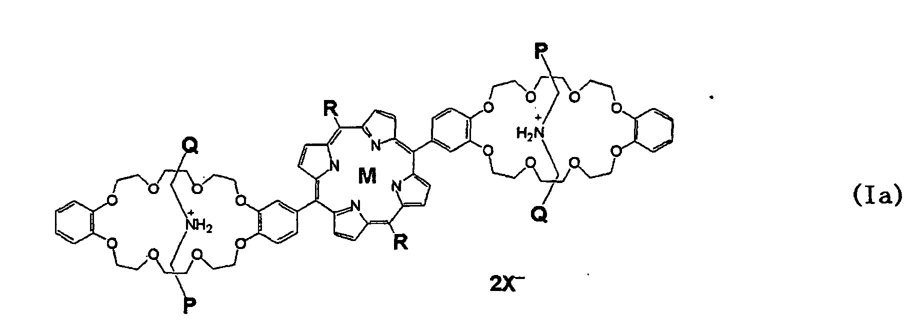 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, Q represents a hydrocarbyl group selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.

12. A process for the preparation of a polymer represented by the following general formula (IIb): 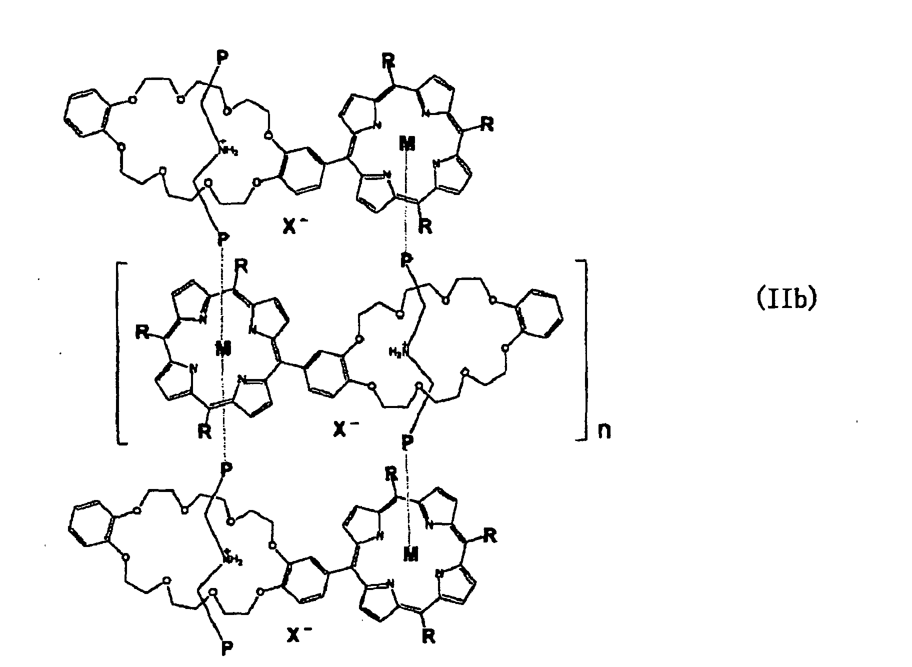 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups, X represents an arbitrary anion atom and n is an integer of 1 or more,
said process comprising polymerizing a monomer represented by the following general formula (Ib): 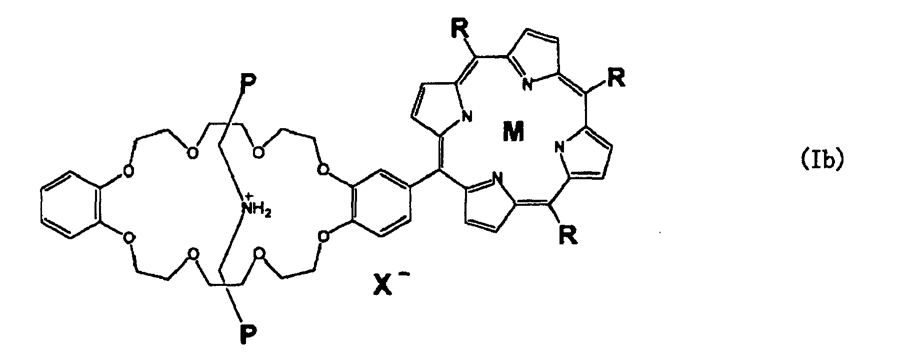 wherein M represents a transition metal coordinatable with the four nitrogen atoms and two additional ligands, P represents a group having, at a terminus thereof through a hydrocarbyl group, a nitrogen atom coordinatable with a metal, said hydrocarbyl group being selected from aliphatic hydrocarbon groups and aromatic hydrocarbon groups, R represents a hydrocarbyl group selected from aliphatic hydrocarbon groups and aromatic hydrocarbon groups and X represents an arbitrary anion atom.
